(19) Europäisches Patentamt
European Patent Office
Office européen des brevets

(11) **EP 1 919 273 B1**

(12) **EUROPEAN PATENT SPECIFICATION**

(45) Date of publication and mention
of the grant of the patent:
**10.10.2018 Bulletin 2018/41**

(21) Application number: **06766215.5**

(22) Date of filing: **06.08.2006**

(51) Int Cl.:
*A61B 5/00* (2006.01)      *A61B 6/03* (2006.01)
*A61B 6/00* (2006.01)      *A61B 8/13* (2006.01)
*A61B 5/103* (2006.01)     *A61B 5/055* (2006.01)
*A61B 5/053* (2006.01)     *A61B 5/01* (2006.01)

(86) International application number:
**PCT/IL2006/000908**

(87) International publication number:
**WO 2007/015255 (08.02.2007 Gazette 2007/06)**

(54) **TISSUE-CHARACTERIZATION PROBE WITH EFFECTIVE SENSOR-TO-TISSUE CONTACT**

GEWEBEKENNZEICHNUNGSFINGER MIT EFFEKTIVEM SENSOR-GEWEBE-KONTAKT

SONDE DE CARACTERISATION DE TISSU A CONTACT EFFICACE DE CAPTEUR A TISSU

(84) Designated Contracting States:
**AT BE BG CH CY CZ DE DK EE ES FI FR GB GR HU IE IS IT LI LT LU LV MC NL PL PT RO SE SI SK TR**

(30) Priority: **04.08.2005 US 196732**
**09.02.2006 US 350102**

(43) Date of publication of application:
**14.05.2008 Bulletin 2008/20**

(73) Proprietor: **DUNE MEDICAL DEVICES LTD.**
**Caesaria 38 900 (IL)**

(72) Inventors:
• **HASHIMSHONY, Dan**
**37808 Givat Ada (IL)**
• **COHEN, Gil**
**93706 Jerusalem (IL)**
• **GELTNER, Iddo**
**46588 Herzlia (IL)**

(74) Representative: **Dennemeyer & Associates S.A.**
**Postfach 70 04 25**
**81304 München (DE)**

(56) References cited:
WO-A2-2006/116091      US-A1- 2002 072 676
US-B1- 6 647 089        US-B2- 6 689 073

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD AND BACKGROUND OF THE INVENTION

The present invention relates to local tissue characterization and, more particularly, to a tissue-characterization probe with effective sensor-to-tissue contact. The probe is further adapted for providing three-dimensional information.
A large number of techniques and sensors are available today for tissue characterization, for example, to determine the presence of abnormal tissue, such as cancerous or pre-cancerous tissue. These may be incorporated into hand-held probes or miniature probes, adapted for insertion into a body lumen or for use in minimally invasive surgery. While the operating principles of different tissue characterization sensors differ, effective contact between the sensor and the tissue is often essential for reliable results. For example, the presence of air bubbles between an ultrasound sensor and the tissue will interfere with ultrasound measurements. Similarly, a liquid layer may interfere with an optical spectroscopy sensor.
The use of suction, for engaging a medical instrument to a tissue, is known. For example, US Patent 5,927,284 to Borst, entitled "A Method and Apparatus for Temporarily Immobilizing a Local Area of Tissue," describes temporarily immobilizing a local area of heart tissue to permit surgery on a coronary vessel in that area without significant deterioration of the pumping function of the beating heart. The local area of heart tissue is immobilized to a degree sufficient to permit minimally invasive or micro-surgery on that area of the heart. A suction device is used to accomplish the immobilization. The suction device is coupled to a source of negative pressure. The suction device has a series of suction ports on one surface. Suction through the device causes suction to be maintained at the ports. The device is shaped to conform to the surface of the heart. Thus, when the device is placed on the surface of the heart and suction is created, the suction through the ports engages the surface of the heart. The suction device is further fixed or immobilized to a stationary object, such as an operating table or a sternal or rib retractor. Thus, the local area of the heart near the suction device is temporarily fixed or immobilized relative to the stationary object while suction is maintained. In this fashion, the coronary artery may be immobilized, even though the heart itself is still beating so that a bypass graft may be performed. In addition, the suction device may be used in either a conventional, open-chest environment or in a minimally-invasive, endoscopic environment.
US Patent 5,727,569 to Benetti, et al., entitled "Surgical Devices for Imposing a Negative Pressure to Fix the Position of Cardiac Tissue During Surgery," also teaches devices and techniques of using a negative (suction) pressure or vacuum, applied through surgical instruments, for fixing the position of a portion of the surface of a beating heart so that a surgical procedure can be more easily performed. The devices apply negative pressure at several points on the outer surface of the heart such that a portion of the heart is fixed in place by the suction imposed through the surgical instruments. Because each device fixes the position of the tissue, and because the instruments remain at a constant distance from the particular portion of the heart where surgery is performed, the device may also serve as a support or platform so that other surgical instruments or devices can be advantageously used at the site. In certain preferred embodiments, the devices described are structured to facilitate the use of additional surgical instruments such that the placement of a negative pressure device permits the surgeon to advantageously manipulate the other instruments during surgery. The negative pressure is preferably imposed through a plurality of ports that may be disposed in a substantially planar surface of the instrument that contacts the cardiac tissue.
Additionally, US Patent 6,728,565 to Wendlandt, entitled "Diagnostic Catheter Using a Vacuum for Tissue Positioning," describes the use of a diagnostic catheter, associated with a vacuum source, for attaching a sensor to a tissue surface. The method includes inserting a catheter with a sensor at its distal end into the body of a patient, applying suction through the catheter, to draw tissue into a predetermined sensing position for the sensor, and analyzing the tissue with the sensor. The degree of vacuum may be adjusted, so that only the required amount of force is used to maintain contact between the sensor or sensors and the tissue being analyzed.
US Patent 6,090,041 to Clark, entitled "Vacuum Actuated Surgical Retractor and Methods," describes a surgical retractor for retracting body tissue or organs, using suction. The surgical retractor includes an end piece adapted for sealing engagement with body tissue, the end piece having at least one suction port therein, the at least one suction port operably linked to at least one vacuum line. Suction supplied to the at least one suction port may be controlled by a vacuum control unit. Retractors may be provided in a range of shapes and sizes, according to the intended application or tissue to be retracted. A method for making a vacuum actuated retractor is disclosed, together with a method for automatically retracting body tissue.
US Patent 6695782 to Ranucci, et al., teaches an ultrasonic tissue ablation device comprising a transversely elongated probe which may be coupled to the device body, the device including an ultrasound energy source and sound conductor, and a method of use of the device and probe for removal of vascular occlusions in blood vessels. A coupling assembly enables incorporation of elongated probes with small cross-sectional lumens such as a catheter guide wire. The probe can be used with acoustic and/or aspiration sheaths to enhance destruction and removal of an occlusion. The horn assembly of the device that contains a sound conducting horn functions as an energy regulator and reservoir for the probe, and precludes loss of probe cavitation energy by its bending or damping within the blood vessel.

US Patent 6,500,112 to Khouri, entitled "Vacuum Dome with Supporting Rim and Rim Cushion," describes the use of vacuum for tissue stretching, to enlarge a soft tissue, for example after a breast surgery, or to correct a deformity. It utilizes a generally rigid dome, capable of withstanding a pressure differential, with a rim cushion underlying the rim of the dome, for supporting the rim against the patient's skin surface. The rim may be generally wider than the dome in order to distribute the attendant forces across a greater surface and avoid tissue damage. A sticky sole underlies the rim cushion and seals the rim cushion to the patient's skin, to thereby preserve the vacuum within the dome. The sticky sole may be any adhesive material and may be achieved through the use of an appropriate material for the rim cushion itself. Unlike the other references, described hereinabove, in US Patent 6,500,112, the vacuum is used for its therapeutic effect, i.e., tissue stretching, to enlarge a soft tissue or to correct a deformity, rather than as means for attaching another instrument.

While all of the aforementioned devices relate to engagement with a tissue, they do not provide the effective contact between the sensor and the tissue itself, which is often essential for reliable results. For example, the presence of air bubbles between an ultrasound tool and the tissue will interfere with ultrasound measurements. Similarly, a liquid layer may interfere with optical spectroscopy. There is thus a need for devices and methods for ensuring effective contact between a tissue-characterization sensor and a tissue, free of air, liquid and foreign matter.

International Patent Application No. WO2006/116091 to Richard J. Davies, forms part of the state of the art under Article 54(3) EPC and discloses a method and system for detecting electrophysiological changes in pre-cancerous and cancerous tissue and epithelium.

## SUMMARY OF THE INVENTION

A device for substance-characterization, is provided, designed for effective sensor-to-substance contact. The device includes an element, having a rigid surface, on which at least one sensor is arranged, and a mechanism for causing a force to be applied to a soft, pliable substance, the line of force being at a sharp angle with the rigid surface, for stretching or stretching and pushing the soft substance against the rigid surface, thus achieving effective contact between the substance and the at least one sensor. In consequence, the accuracy of the sensing is improved. In accordance with another embodiment, a plurality of sensors is employed, arranged along a curved element, for providing three-dimensional information regarding the substance, for example, by small-scale computerized tomography. Alternatively, the device comprises a structure; a first mechanism, configured for exerting a first force on a substance, for fixing the substance to the structure, so as to substantially immobilize the substance; and a second mechanism, configured for pressing at least one sensor against an external surface of the immobilized substance, thereby exerting a second force on the immobilized substance, wherein at least a component of the first force is in opposition to at least a component of the second force, forcing the immobilized substance against the at least one sensor, and forcing the at least one sensor against the immobilized substance, bringing about an effective contact between the at least one sensor and the immobilized substance. The substance may be tissue, ex-vivo or in situ, or a biopsy sample. Alternatively, it may be another soft, pliable substance, such as hydrogel or an elastomer.

In accordance with an aspect of the invention, there is provided a device and method in accordance with the appended claims.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and are not intended to be limiting.

## BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

Figures 1A-I schematically illustrate longitudinal cross-sections of devices for effective sensor-to-tissue contact, in accordance with some embodiments of the present invention;

Figures 1J-M schematically illustrate situations of potentially poor contact between a tissue and a device, as seen in a longitudinal cross-sectional or top view, in accordance with the understandings of the present invention;

Figure 1N schematically illustrates effective contact between a tissue and a device, as seen in a longitudinal cross-section, in accordance with the embodiments of the present invention;

Figures 1O - 1V schematically illustrate various transverse and longitudinal cross-sections of a device, in accordance with some embodiments of the present invention.

Figures 2A - 2E schematically illustrate, in longitudinal cross-sectional and perspective views, a probe for tissue characterization, constructed in accordance with the present invention;

Figure 3 schematically illustrates a system for tissue characterization, in accordance with some embodiments of the present invention;

Figures 4A and 4B schematically illustrate a first arrangement of the sensors in the device, constructed in accordance with some embodiments of the present invention;

Figure 5 is a flowchart illustrating a method of tissue characterization, in accordance with some embodiments of the present invention;

Figures 6A-F schematically illustrate arrangements of the sensors in the device for providing three-dimensional information, in accordance with further embodiments of the present invention;

Figures 6G-N schematically illustrate elements for providing three-dimensional information, in accordance with further embodiments of the present invention; Figure 6-O is a flowchart illustrating a method of tissue characterization in three-dimensions, with small-scale computerized tomography, in accordance with some embodiments of the present invention;

Figure 7A is a side cross-sectional view of a device for forming effective sensor-to-tissue contact, according to an embodiment not claimed herein;

Figure 7B is a diagram which schematically illustrates the forces at a tissue-sensor interface, in accordance with an embodiment not claimed herein;

Figure 7C schematically illustrates a longitudinal cross-section of the device of Figures 7A-B, for forming effective sensor-to-tissue contact, in accordance with an embodiment not claimed herein;

Figures 8A-C schematically illustrate the proximal end of a device for forming effective sensor-to-tissue contact, during three steps in the operation of the device, in accordance with an embodiment not claimed herein;

Figures 9A-C are side and top cross-sectional views of a first mechanism of a device according to an embodiment not claimed herein;

Figures 10A-D are side and top cross-sectional views of a first mechanism of a device, according to another embodiment not claimed herein;

Figures 11A-B are side cross-sectional views of a second mechanism, for forming effective sensor-to-tissue contact, during two steps in the operation of the device, according to an embodiment not claimed herein;

Figures 12A-B are side cross-sectional views of the second portion of a device for tissue characterization, showing a second mechanism for forming effective sensor-to-tissue contact, according to further embodiments not claimed herein;

Figures 13A-D schematically illustrate means for controlling the travel of the piston, according to two alternative embodiments not claimed herein;

Figures 14A-D schematically illustrate a coordinate system and various geometries for the proximal end of the sensor, in accordance with some embodiments not claimed herein;

Figures 15A-D schematically illustrate a prototype of a device for tissue characterization, in accordance with an embodiment not claimed herein;

Figures 16A-D schematically illustrate a prototype of a device for tissue characterization, in accordance with another embodiment not claimed herein;

Figures 17A-E schematically illustrate various embodiments of sensors for use with the device of the present invention;

Figures 18A-C schematically illustrate another configuration of a device for effective contact, during three steps in the operation of the device, in accordance with another embodiment not claimed herein;

Figure 19 schematically illustrates a system for tissue characterization, in accordance with an embodiment of the present invention;

Figures 20A-C schematically illustrate another configuration of a device for effective contact, in accordance with another embodiment of the present invention;

Figures 21A-B schematically illustrate configurations of a device with several types of sensors, in accordance with embodiments of the present invention;

Figure 22 schematically illustrates a first sensor construction, in accordance with some embodiments of the present invention;

Figure 23 schematically illustrates a second sensor construction, in accordance with some embodiments of the present invention;

Figures 24A-B schematically illustrate optical sensor constructions, in accordance with some embodiments of the present invention;

Figures 25A - 25D schematically illustrate an endoscopic probe 400, in accordance with embodiments of the present invention; and;

Figures 26A and 26B schematically illustrate the probe as an extracorporeal probe, in an examination station, in accordance with embodiments of the present invention.

## DESCRIPTION OF THE PREFERRED EMBODIMENTS

A device for substance-characterization, designed for effective sensor-to-substance contact, includes an element, having a rigid surface, on which at least one sensor is arranged, and a mechanism for causing a force to be applied to a soft, pliable substance, the line of force being at a sharp angle with the rigid surface, for stretching or stretching and pushing the soft substance against the rigid surface, thus achieving effective contact between the substance and the at least one sensor. In consequence, the accuracy of the sensing is improved. In accordance with another embodiment, a plurality of sensors is employed, arranged along a curved element, for providing three-dimensional information regarding the substance, for example, by small-scale computerized tomography. Alternatively, the device comprises a structure; a first mechanism, configured for exerting a first force on a substance, for fixing the substance to the structure, so as to substantially immobilize the substance; and a second mechanism, configured for pressing at least one sensor against an external surface of the immobilized substance, thereby exerting a second force on the immobilized substance, wherein at least a component of the first force is in opposition to at least a component of the second force, forcing the immobilized substance against the at least one sensor, and forcing the at least one sensor against the immobilized substance, bringing about an effective contact between the at least one sensor and the immobilized substance. The substance may be tissue, ex-vivo or in situ, or a biopsy sample. Alternatively, it may be another soft, pliable substance, such as hydrogel or an elastomer.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

The principles and operation of the device for tissue-characterization, according to some embodiments of the present invention, may be better understood with reference to the drawings and accompanying descriptions.

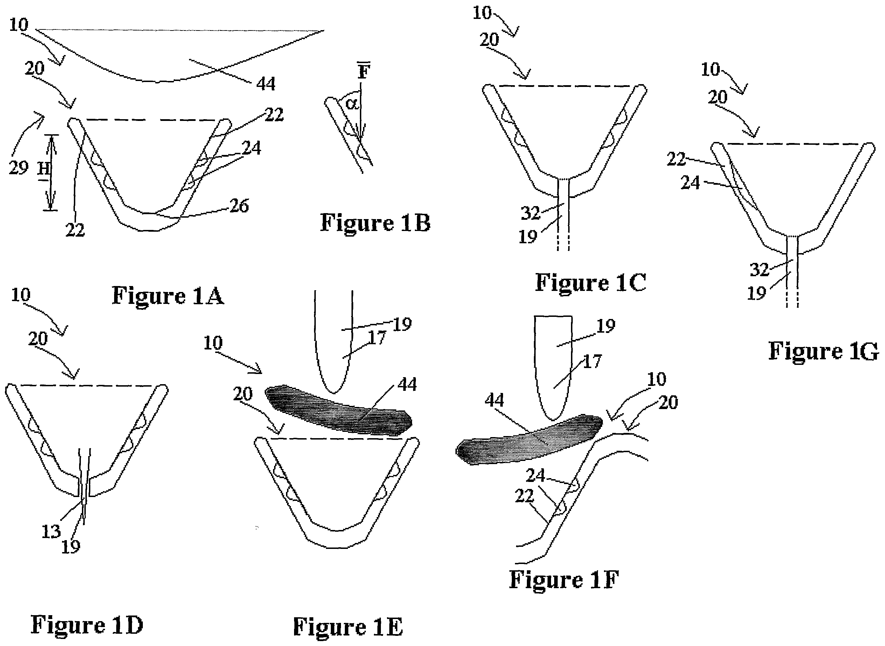

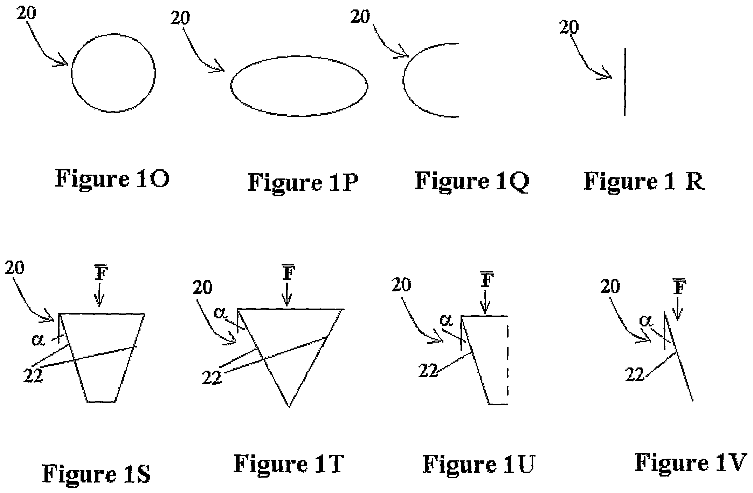

Referring now to the drawings, Figures 1A-H schematically illustrate a longitudinal cross-section of a device 10, for effective sensor-to-tissue contact, in accordance with some embodiments of the present invention.

Accordingly, the device 10 includes an element 20, at a proximal end 29 thereof, configured to make contact with a tissue 44. As seen in Figures 1A - 1E, and IG the element 20 may be substantially configured as a cone, having a base 26.

However, as seen in Figure 1F, other shapes may similarly be used. Some of these are described hereinbelow, in conjunction with Figures 1O - 1V.

Preferably, the tissue 44 is a soft tissue, such as the tissue of muscle, skin, fat, internal organs, internal interfaces and the like, which generally yields under pressure.

It will be appreciated that the tissue characterization may be conducted for tissue in situ, tissue ex-vivo, or a to a biopsy sample.

In accordance with the present invention, tissue in situ refers to tissue in place, that is, attached to the body.

Further in accordance with the present invention, tissue ex-vivo refers to tissue that has been removed, for example, during an operation.

Still further in accordance with the present invention, a biopsy sample refers to a small portion of a tissue that has been removed, in order to determine a need for further action, for example an operation.

As such, a biopsy sample may relate to a small portion of a tumor, removed in order to determine if the tumor is malignant, while tissue ex-vivo may relate to the whole tumor, removed during surgery, and examined for example, to evaluate if all malignant portions are surrounded by a clean margin of healthy tissue, so that no malignancy has remained in the body.

Nevertheless, it will be appreciated that the distinction between a biopsy sample and tissue ex-vivo is fine, and there is a certain degree of overlap between them.

It will be appreciated that the device 10 may be used for other pliable materials, such as gels, hydrogels, or elastomers.

Preferably, the element 20 includes a section of a height H, the section having a linear cross section, and forming a rigid surface 22. The contact of the element 20 with the tissue 44 is made along the rigid surface 22 of the preferably linear cross section of element 20.

Additionally, the device 10 includes at least one sensor 24, associated with the rigid surface 22. A plurality of sensors 24 may be employed. For example, while the device of Fig. 1G includes a single sensor 24, the device of each of Figs. 1A-F includes a plurality of sensors. The at least one sensor 24 may be embedded within or mounted on the rigid surface 22.

Furthermore, the device 10 includes a mechanism 19, adapted for applying a force F to the tissue 44, the line of force being at a sharp angle $\alpha$ to the rigid surface 22, for stretching or stretching and pushing the tissue 44 against the rigid surface 22, thus achieving effective contact between the tissue 44 and the rigid surface 22. In consequence, effective contact between the tissue 44 and the at least one sensor 24 is formed, and the accuracy of the sensing is improved.

Figure 1B illustrates the angular relationship between the force F and the rigid surface 22. Intuitively, one may observe that, were the force **F** parallel with or perpendicular to the rigid surface 22, the desired stretching of the tissue 44 against the rigid surface 22 would not happen. The sharp angle $\alpha$ between the line of force **F** and the rigid surface 22 is essential for the practice of the present invention. Preferably, the sharp angle $\alpha$ is between about 30 degrees and about 60 degrees, yet other values of sharp angles may also be acceptable.

Additionally, one may observe that, were the cross-sectional profile of the surface 22 curved, the stretching of the tissue 44 against the rigid surface 22 would not be uniform along the section of height H. Thus, in accordance with the preferred embodiment of the present invention, the surface 22 has a linear cross-sectional profile.

As seen in Figure 1C, the mechanism 19, which provides the force F, may include a vacuum source 32, for essentially sucking the tissue 44 into the element 20.Alternatively, as seen in Figure 1D, the mechanism 19 may be a mechanical tool, for example, a tweezer-like tool 13, for pulling the tissue 44 into the element 20. Alternatively, as seen in Figures 1E and 1F, the mechanism 19 may be another mechanical tool, for example, a mallet-like tool 17, for pressing the tissue 44 into the element 20.

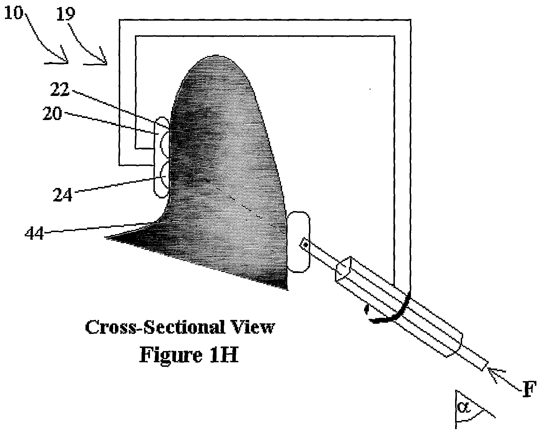

Figure 1H schematically illustrates still another example of a mechanism 19 for applying the force F to the tissue 44, at a sharp angle $\alpha$ to the rigid surface 22, wherein the rigid surface 22 may be a flat plate. The mechanism 19 may be, for example, a piston-cylinder configuration, arranged at the angle $\alpha$ to the flat plate, operative as the element 20, and having the rigid surface 22, in which the sensors 24 are embedded.

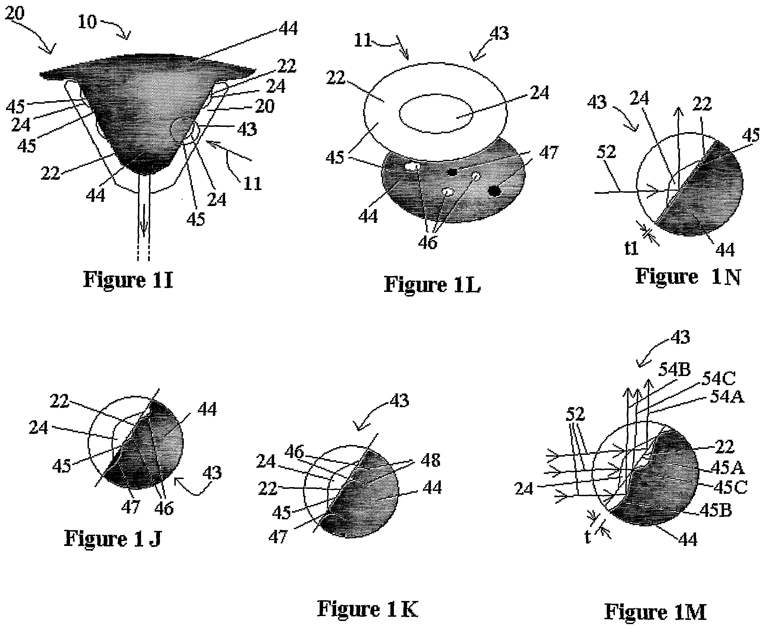

Referring further to the drawings, Figures 1I-M schematically illustrate situations of potentially poor contact between the tissue 44 and the rigid surface 22 of the element 20, in which the sensors 24 are embedded in the device 10, in accordance with the understandings of the present invention. In contrast, Figure 1N schematically illustrates effective contact between the tissue and the device 10, as seen in the longitudinal cross-section, in accordance with embodiments of the present invention.

Figure 1I is a cross-sectional view of an interface 45, between the rigid surface 22 of the element 20 and the at least one sensor 24 and the tissue 44. A section 43 is marked and enlarged in Figures 1J - IN, discussed hereinbelow.

Figure 1J provides a cross-sectional view of the interface 45, at the section 43, showing bubbles 46 of air or fluids and/or inclusions 47 of foreign matter, which reduce and otherwise interfere with the contact area between the tissue 44 and the at least one sensor 24, along the interface 45. Figure 1K also provides the cross-sectional view of the interface 45, at the section 43, showing that tissue folds 48, possibly with bubbles 46 and/or inclusions 47, may also reduce and otherwise interfere with the contact area between the tissue 44 and the at least one sensor 24, along the interface 45.Figure 1L provides a view of the interface 45, at the section 43, from the direction of an arrow 11 of figure 1I, showing the bubbles 46 and the inclusions 47, interfering with the contact at the interface 45.

With regard to the contact area between the tissue 44 and the at least one sensor 24, the following terms may be defined:

- Actual contact area, A(actual), is the actual contact area between the rigid surface 22 and the tissue 44;
- Overall contact area, A(interface), is the whole area of the interface 45; and
- Bubble and inclusions area, A(bubbles and inclusions), is an area covered by bubbles 46 of air and/(or fluid, and/or by inclusions 47 of foreign matter.

One may then calculate the actual contact area and a contact level, as follows:

$$[1] \qquad A(actual) = A(interface) - A(bubbles\ and\ inclusions),$$

and,

$$[2] \quad \text{Contact Level} = \frac{A(\text{actual})}{A(\text{interface})}.$$

Furthermore, one can quantify the effect of the bubbles 46 and the inclusions 47, and evaluate if the interface 45 is acceptable for tissue characterization, with a given sensor.

Embodiments of the present invention are aimed at achieving *effective contact,* which is defined, for the purposes of the present invention, as a contact level of at least 95%. Preferably, the contact level is greater than 98%. More preferably, the contact level is at least 99.5%, and even at least 99.8%.

Figure 1M provides the cross-sectional view of the interface 45, at the section 43, with reference to irradiative sensor 24, showing a situation where the edge surface of the irradiative sensor 24 and the external-most surface of the tissue 44 are slightly apart, by an average distance t so that, in effect, there are three interfaces 45A, 45B, and 45C, which may operate as three distinct reflective surfaces to incoming radiation 52. The first interface, 45A, is at the edge surface of the at least one sensor 24 (which is essentially the same as the rigid surface 22); the second interface, 45B, is at the external-most surface of the tissue 44; and the third interface, 45C, is between the edge surface of the at least one sensor 24 and the external-most surface of the tissue 44, when there is substantially complete contact. This effect may be important for radiation of a wavelength $\lambda$, for which the average distance t and the radiation wavelength $\lambda$ are of the same order of magnitude and, in consequence, three reflections 54A, 54B, and 54C may be observed, from the interfaces 45A, 45B, 45C, respectively, rather than the single reflection 54C, of the joint interface.

In contrast with Figures 1J - 1M, Figure 1N schematically illustrates effective sensor-to-tissue contact, in accordance with some embodiments of the present invention. Accordingly, the interface 45 is substantially free of bubbles 46, foreign inclusions 47, and tissue folds 48, leading to *effective contact* between the tissue 44-and the at least one sensor 24, the effective contact being defined as a contact level of at least 95%, preferably, at least 98%, and more preferably, at least 99.5% and even at least 99.8%.

Additionally, in accordance with embodiments of the present invention, which relate to sensors operating with a wavelength $\lambda$, the effective contact may be further defined as a contact, for which the relationship between the wavelength $\lambda$ and an average distance, t1, after achieving effective contact (see Figure IN), is such that $t1 < \lambda/3$, and preferably, $t1 < \lambda/10$, and more preferably, $t1 < \lambda/100$.

Additionally or alternatively, the effective contact may be defined in absolute terms. Accordingly, the average distance t1 is less than 500 Angstroms, preferably the average distance t1 is less than 50 Angstroms, and more preferably, the average distance t1 is less than 5 Angstroms.

Referring further to the drawings, Figures 1O - 1V schematically illustrate various transverse and longitudinal cross sections of the element 20, in accordance with some embodiments of the present invention. All are associated with the rigid surface 22 of the linear cross section, arranged at the angle $\alpha$ to the line of force F.

Accordingly, the transverse cross-sectional configuration of the element 20 may be a circle (Figure 1O), an ellipse (Figure 1P), an arc (Figure 1Q), or a line associated with a flat plate (Figure 1R), while the longitudinal cross-sectional configuration may be a trapezoid (Figure IS), a triangle (Figure IT), a section of a trapezoid or triangle (Figure 1U), or a line (Figure 1V). Thus, the overall shape of the element 20 may be substantially a cone, with a circular or an elliptical cross-section, with a base or with no base, a section of a cone, or a flat plate.

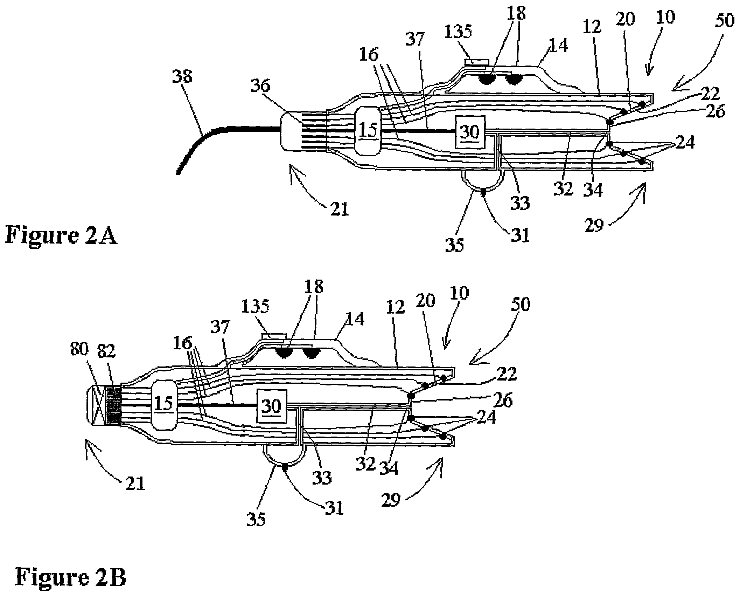

Referring further to the drawings, Figures 2A - 2E schematically illustrate a probe 50 for tissue characterization, constructed in accordance with the device 10 of the present invention. The probe 50 includes a housing 12, which includes the device 10 with the element 20 having a conical configuration, as described hereinabove, in conjunction with Figures 1A - 1V, and at least one sensor 24.

In accordance with the present embodiment of Figures 2A - 2E, the probe 50 is an extracorporeal probe, adapted as a hand-held probe, via a gripping handle 14.

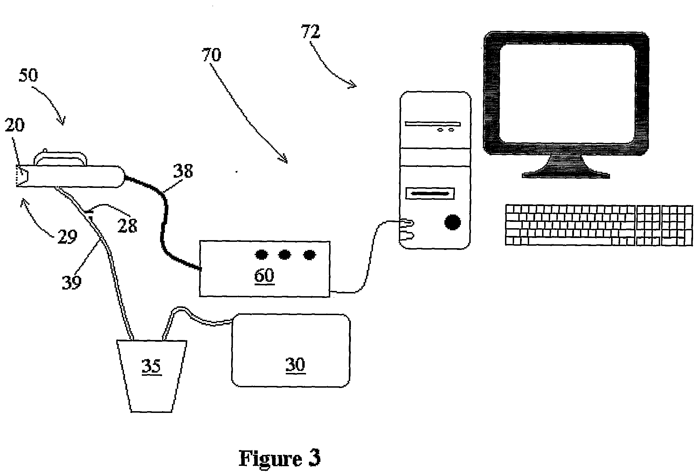

As seen in Figure 2A, a longitudinal cross-sectional view preferably at a distal end 21 of the probe 50, a signal communication architecture 16 of at least one signal communication line leads from the at least one sensor 24 to a connector 36, associated with a cable 38, which provides power and signal communication with a signal-generating and analyzing unit 60 described hereinbelow in conjunction with Figure 3. A plurality of sensors 24 and a plurality of signal communication lines forming the signal communication architecture 16 may be employed. The at least one signal communication line may be a transmission line, for example, a coaxial cable or an optical fiber.

As seen in Figure 2B, in the longitudinal cross-sectional view, a battery 80 and a transceiver 82 may be provided at the distal end 21, for wireless operation of the probe 50 and for wireless communication with the signal-generation and analyzer unit 60 (Fig. 3). It will be appreciated that the battery 80 may be rechargeable.

The probe 50 may further include a pump 30, receiving power via a power line 37 and in fluid communication with the element 20, for providing suction thereto via an orifice 34 and a channel 32 in the element 20.

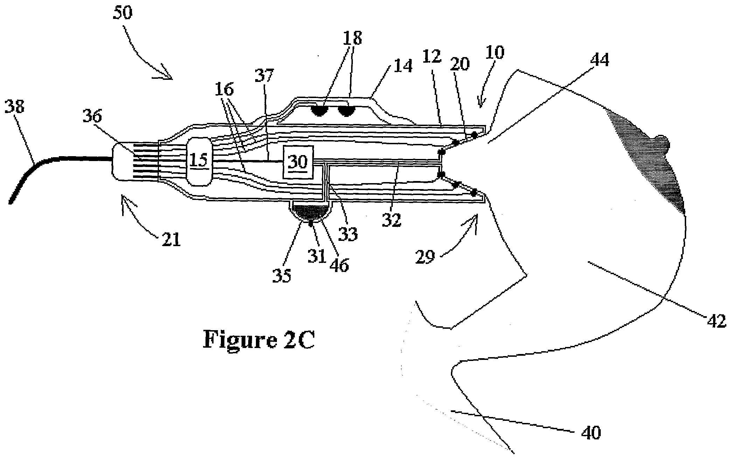

As seen in Figure 2C, in the longitudinal cross-sectional view, the probe 50 may be used for characterizing the soft tissue 44, for example, of a breast 42 of a body 40, during open surgery. When suction is applied to the soft tissue 44, it is drawn into the element 20, maintaining effective contact with the at least one sensor 24. Additionally, during surgery, fluids 46 may be drawn as well and directed by a channel 33 to a fluid trap 35, which may be emptied via a valve 31.

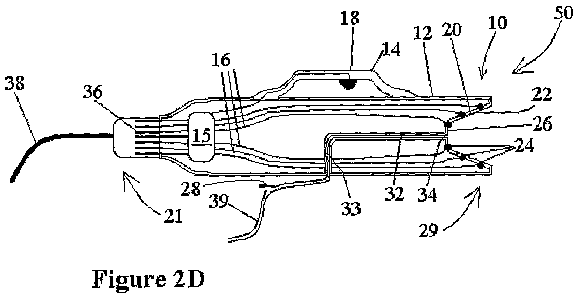

As seen in Figure 2D, in the longitudinal cross-sectional view, a vacuum source (not shown) external to the probe 50 may be used, via a vacuum line 39. A sealing flap 28, along the vacuum line 39, may close when a vacuum is applied, creating suction in the element 20. The vacuum line 39 may connect with a pump 30 and the fluid trap 35. Alternatively, vacuum line 39 and pump 30 may be external to the probe 50, as described hereinbelow, in conjunction with Figure 3.

As seen in Figures 2A - 2D, the probe 50 may further include at least one control switch 18, for initiating the measurement by the at least one sensor 24, and for controlling the pump 30 (Figures 2A - 2C). If desired, two control switches 18 may be provided, one for operating the sensor or sensors 24 and the other for operating the pump 30.

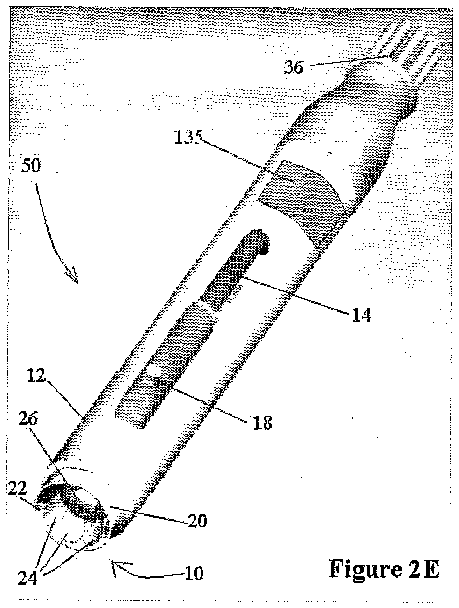

Additionally, the probe 50 may include an indicator 135, for example, a light, an LED display, or a display screen 135 (seen in Figures 2A, 2B, and 2E).

The at least one control switch 18 and the indicator 135 may be used for manipulating the probe 50 and for optimizing the performance of the probe 50 and the sensors 24, for example, for providing feedback which is easily seen the operator, in real time.

A junction 15 may be provided as a switching station, communicating with the at least one control switch 18, the indicator 135, the signal communication architecture 16, and the pump power line 37. It will be appreciated that the at least one sensor 24 may have a "standby" setting, for use prior to operation. Figure 2E provides a perspective view of the probe 50, according to an embodiment of the present invention.

Referring further to the drawings, Figure 3 schematically illustrates a system 70 for tissue characterization, in accordance with some embodiments of the present invention. Preferably, the system 70 includes the probe 50, having the device 10 with the element 20 and at least one sensor 24, designed in accordance with some embodiments of the present invention. The probe 50 may be in fluid communication with an external fluid trap 35 and an external pump 30. Alternatively, these may be built into the probe 50.

Preferably, a signal generating and analyzing unit 60 communicates with the at least one sensor 24, either via a cable 38 or in a wireless manner, as known in the art. The signal generating and analyzing unit 60 may include a built-in computer, or may communicate with a computer station 72, which analyzes measurements performed by the probe 50. Alternatively, a miniaturized signal generating and analyzing unit 60 and possibly also a microcomputer (not shown) may be built into the probe 50. It will be appreciated that separate units may be employed in place of the single signal generating and analyzing unit 60. Additionally, some sensors are passive and do not require signal generators. For example, a temperature sensor, or a radioactive-emission sensor do not require signal generators.

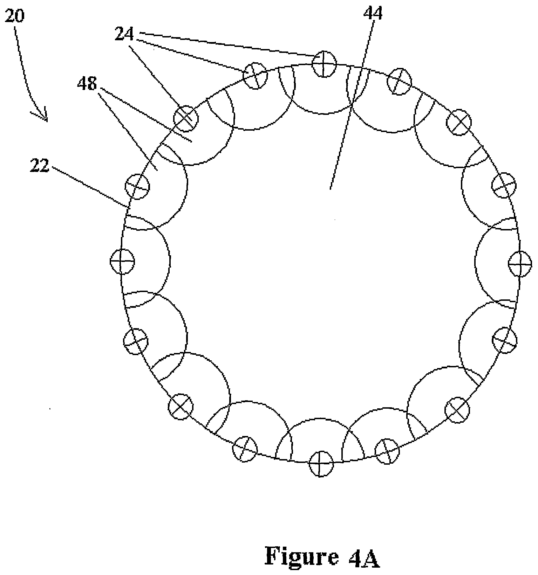

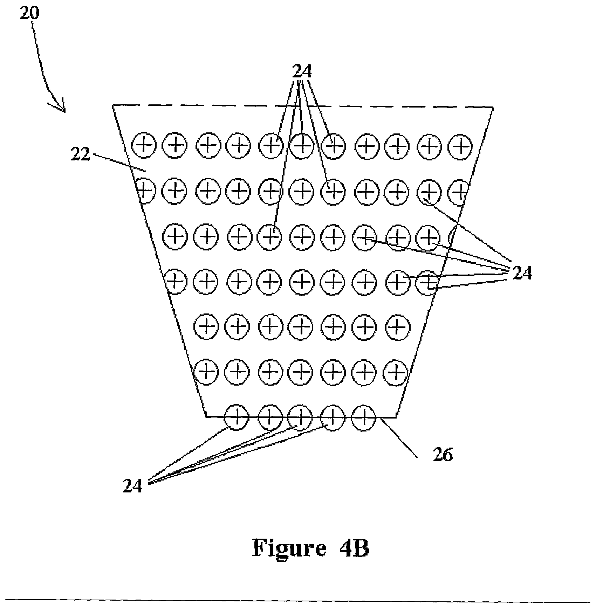

Referring further to the drawings, Figures 4A and 4B schematically illustrate a first arrangement of the at least one sensor 24 in the device 10, in accordance with some embodiments of the present invention. As discussed above, the element 20 may be configured substantially as a cone or as any other suitable shape having the rigid surface 22, and the at least one sensor 24 is embedded within or mounted on the rigid surface 22 of the element 20.

As seen in Figure 4A, each of the sensors 24 characterizes the tissue 44 within a generally hemispherical volume 48, adjacent thereto. As seen in Figure 4B, where the element 20 is configured as a truncated cone, the sensors 24 may also be arranged along the base 26. The at least one sensor 24 may be an irradiative sensor such as an optical sensor, an X-ray sensor, an RF sensor, an MW sensor, an infrared thermography sensor, or an ultrasound sensor. Alternatively, the at least one sensor 24 may be an MR sensor; an impedance sensor; a temperature sensor; a biosensor; a chemical sensor; a radioactive-emission sensor; a mechanical sensor; a nonradiative RF sensor, for example, as taught by commonly owned US Patent Application 60/665,842, filed on March 29, 2005, or any other suitable tissue characterization sensor.

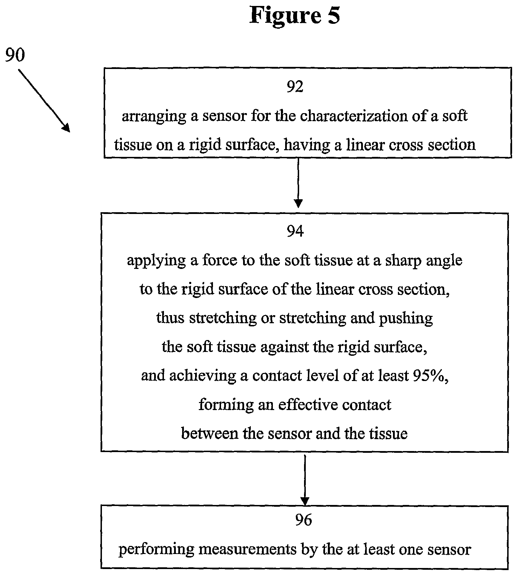

Figure 5 schematically illustrates a method 90 for soft tissue characterization, by improving the contact level between a soft tissue and at least one sensor, in accordance with some embodiments of the present invention. The method 90 includes:

in step 92: arranging at least one sensor for the characterization of a soft tissue on a rigid surface, having a linear cross sec;
in step 94: applying a force to the soft tissue at a sharp angle to the rigid surface, thus stretching or stretching and pushing the soft tissue against the rigid surface, and achieving *effective contact* between the sensor and the tissue; and
in step 96: performing measurements with the at least one sensor.

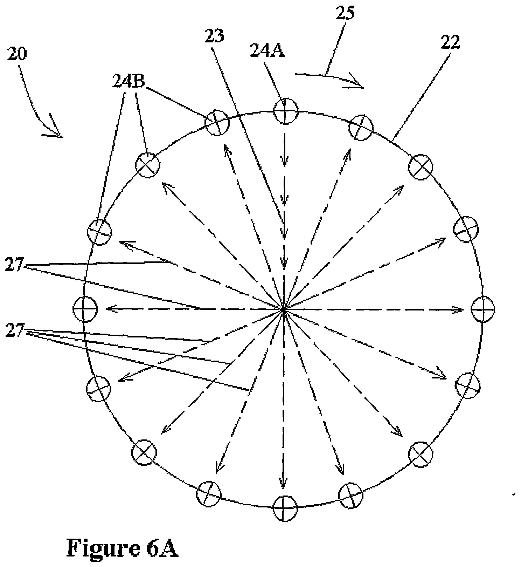

Referring further to the drawings, Figures 6A - 6F schematically illustrate arrangements of a plurality of the sensors 24 in the device 10, for yielding three-dimensional information, for example, by small-scale computerized tomography, in accordance with some embodiments of the present invention.

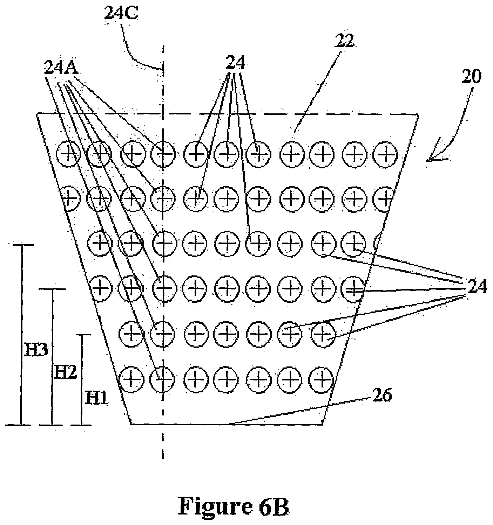

As seen in Figures 6A and 6B, the element 20 may be formed as a circular structure, such as a truncated cone, with a plurality of the sensors 24, preferably arranged in circles around the internal circumference, embedded within or mounted on the rigid surface 22, at different heights therealong, for example, at HI, H2, and H3. Preferably, the sensors 24 of each circle are substantially aligned, vertically, so as to define lines along the rigid surface 22, such as the line 24C (Figure 6B).

The sensors 24 are adapted for small-scale computerized tomography, which may be transmission small-scale computerized tomography, reflection small-scale computerized tomography, or a combination of the two. Preferably, each of the sensors 24 around the circumference, in turn, operates as a transmitting sensor 24A, sending out a signal 23, while the other sensors 24 operate as receiving sensors 24B, receiving signals 27 which may be transmitted, reflected, or a combination of transmitted and reflected. The position of the transmitting sensor 24A may change, for example by rotation, in a direction of an arrow 25. Alternatively, the position of the transmitting sensor 24A may change in another fashion, for example, randomly. In accordance with an embodiment of the present invention, the transmitting sensors 24A are aligned along the line 24C, so as to image "slices of tissue." It will be appreciated that other arrangements are similarly possible. For example, two or more sensors 24 in a circle may operate as transmitters, or as transmitters and receivers, at a given time.

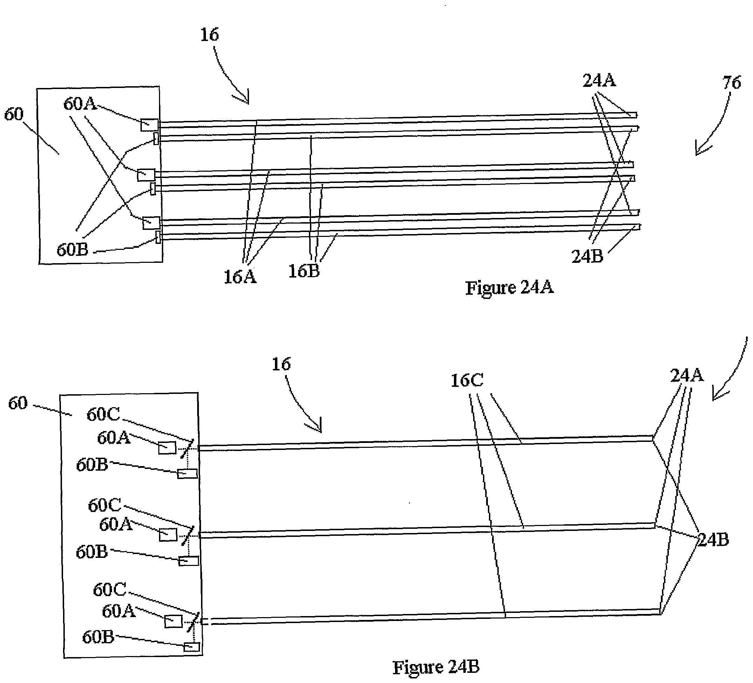

It will be appreciated that, depending on the modality, the transmitting sensor may also operate as a receiving sensor. For example, an ultrasound transducer may operate both as a transmitter and receiver. Similarly, an optical-fiber end may operate as both a transmitter and receiver, for example, as illustrated in conjunction with Figure 24B, hereinbelow. For x-ray CT, dedicated transmitters and receivers may be used.

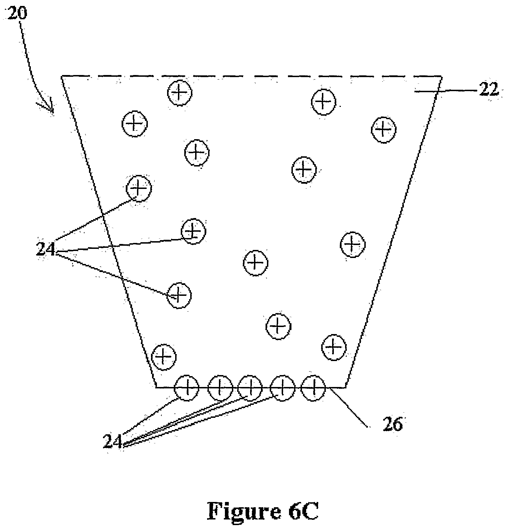

As seen in Figure 6C, the sensors 24 may be randomly spread, and any one sensor 24 may operate as a transmitting sensor, or as a transmitting and receiving sensor, at any one time. The associated algorithm provides the three-dimensional information for the specific arrangement.

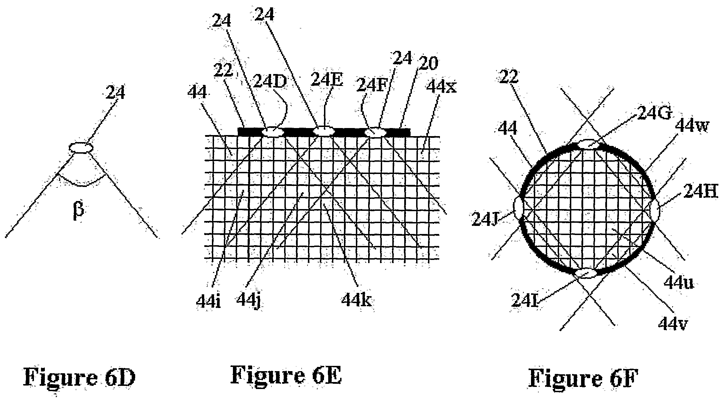

Figures 6D - 6F illustrate configurations that may be used to provide a three dimensional image of a tissue voxel of the tissue 44. As seen in Figure 6D, the sensor 24 has a viewing angle $\beta$. As seen in Figure 6E, when several sensors 24, such as sensor 24D, 24E, and 24F, are arranged along the element 20 formed as a flat plate, the following may be observed:

The tissue voxel 44x is not viewed by any of the sensors; the tissue voxel 44i is viewed only by the sensor 24D; the tissue voxel 44j is viewed by both the sensors 24D and 24E; and the tissue voxel 44k is viewed by the three sensors 24D, 24E and 24F. As illustrated, some three dimensional information may be obtained for the voxels 44j and 44k.

Alternatively, as seen in Figure 6F, when several sensors 24, such as sensors 24G, 24H, 241, and 24J, are arranged along the element 20 formed as a cone or a cylinder, the following may be observed:

the tissue voxel 44u is viewed by all four sensors 24G, 24H, 241, and 24J;
the tissue voxel 44v is viewed by the three sensors 24G, 24H, and 24J; and
the tissue voxel 44w is viewed by the two sensors 241 and 24J.

Thus, in the configuration of Figure 6F, some three dimensional information may be obtained for all the tissue voxels.

Naturally, a rigid surface having a shape with a curvature, such as a circular or elliptical arrangement or a section thereof is preferred to the flat plate arrangement. Nonetheless, the flat plate arrangement does yield some three-dimensional information, and is within the scope of the present invention.

It will be appreciated that, while effective contact is highly desirable, the three-dimensional information may be achieved also without effective contact, thus without the mechanism for applying the force to the tissue, with the line of force at the sharp angle $\alpha$ to the rigid surface 22, as described in conjunction with Figures 1A - 1V. Hence shapes that do not meet this criterion may nonetheless be used for small-scale computerized tomography.

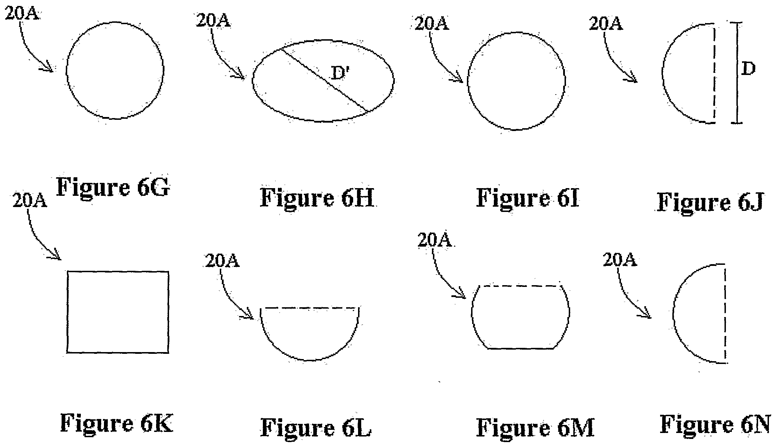

While the shapes illustrated in Figures 1O, 1P, 1Q, IS, IT, and 1U are the most preferred, since they provide both effective contact and curvature, other shapes may also be used, as illustrated in Figures 6G - 6N.

Figures 6G - 6N schematically illustrate elements 20A, shaped with a curvature, for providing three-dimensional information, but without necessarily providing effective contact. These include elements shaped as a cylinder (Figures 6G and 6K), an ellipsoid (Figures 6H and 6L), a portion of a sphere (Figures 6J and 6N), and a barrel (Figures 6I and 6M).

Preferably the curved surface has a diameter D (Figure 6J), or a diameter equivalent D' (Figure 6H), which is less than about 10 cm. The diameter may be less than 5 cm, less than 2 cm, or even less than 1 cm.

The sensors of Figures 6A - 6N for providing three-dimensional information may be irradiative sensors, such as optical sensors, X-ray sensors, RF sensors, MW sensors, infrared thermography sensors, and ultrasound sensors. Alternatively, the sensors may be mechanical sensors, MR sensors, impedance sensors, nonirradiative RF sensors, radioactive-emission sensors arranged for SPECT, radioactive-emission sensors arranged for PET, and/or any other sensors suitable for tissue characterization. It will be appreciated that other sensors may be used, for example, biosensors or chemical sensors, for providing surface information at different points along the tissue 44, without providing volumetric three-dimensional information.

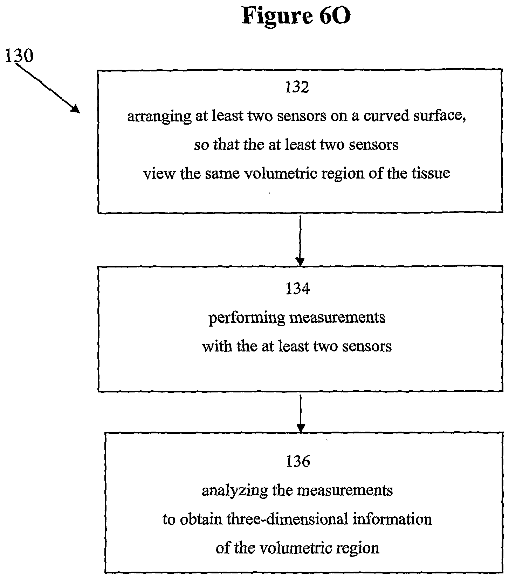

Figure 6-O is a flowchart illustrating a method 130 of tissue characterization with small-scale computerized tomography, for obtaining three-dimensional information of a volumetric region of the tissue, in accordance with some embodiments of the present invention. The method 130 includes:

in step 132: arranging at least two sensors for tissue characterization on a curved surface, so that the at least two sensors view the same volumetric region of the tissue;
in step 134: performing measurements with the at least two sensors; and
in step 136: analyzing the measurements to obtain three-dimensional information of the volumetric region.

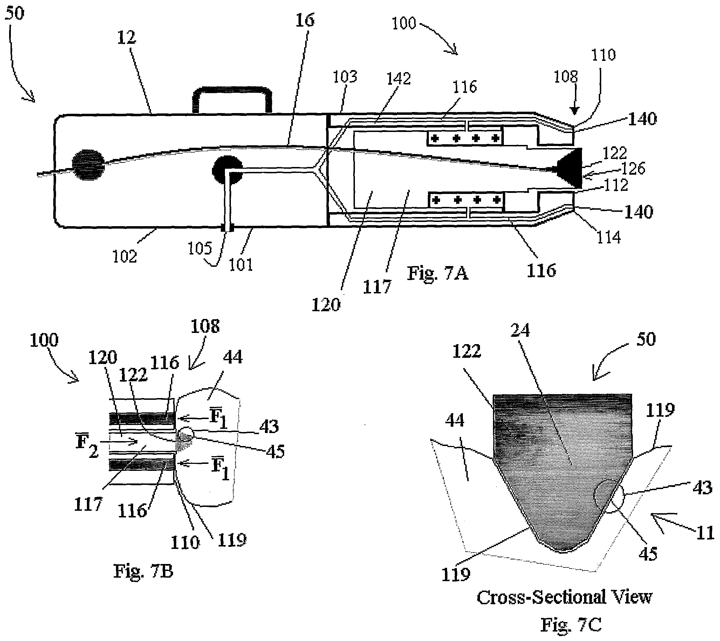

Figures 7A to 18C refer to embodiments not claimed herein, but whose description is considered beneficial for an understanding of the embodiments that are claimed.

Reference is now made to Figures 7A-C, which schematically illustrate the principles of a device 100 for tissue characterization, with effective sensor-to-tissue contact.

Figure 7A is a side cross-sectional view of a device 100, for forming effective sensor-to-tissue contact, to improve tissue characterization. Device 100 comprises:

i. a structure 101;
ii. a first mechanism 116, associated with structure 101 and configured for exerting a first force on a tissue 44, for fixing tissue 44 to structure 101, so as to substantially immobilize the tissue 44; and
iii. a second mechanism 117, associated with structure 101, configured for pressing a sensor 122 against the surface of the immobilized tissue, thereby exerting a second force on the immobilized tissue,

wherein a component of the first force is in opposition to a component of the second force, forcing immobilized tissue against sensor 122, and forcing sensor 122 against the immobilized tissue, bringing about an effective contact between sensor 122 and the immobilized tissue.

In accordance with the present embodiment, structure 101 includes a first portion 102, adapted for hand gripping, and a second portion 103, having a proximal end 108, with respect to the tissue.

The first portion 102 and the second portion 103, together, form the housing 12 of the probe 50. The probe 50 further includes the signal communication architecture 16.

Proximal end 108 has a frame 110, preferably formed as a ring, of an inner diameter 112 and an outer diameter 114. Frame 110 is associated with a first mechanism 116, such as a suction-providing mechanism, designed for attaching the tissue to frame 110.

In accordance with the present embodiment, the first mechanism 116 is formed of a duct system 142 (Figure 7A), having orifices 140, the at the proximal end 108, through which negative pressure attaches tissue 44 to frame 110 by suction. A vacuum outlet 105 is adapted for connecting the duct system 142 to the pump.

Additionally, device 100 includes second mechanism 117, such as a piston 120, within second portion 103. Piston 120 includes sensor 122, at a proximal end 126. Piston 120 is selectively configured for deployed and retracted positions. For deployment, it slides towards and presses against tissue 44, which is held fast by first mechanism 116, thus achieving effective sensor-to-tissue contact between sensor 122 and tissue 44.

It should be understood that, while each of the embodiments as illustrated in the drawings appears to be provided with a single piston sensor 122, if desired, any of the embodiments may be provided with a plurality of piston sensors. Similarly, where the discussion of some of the embodiments may include mention of a plurality of piston sensors, it is to be understood that, if desired, a single piston sensor may alternatively be provided.

Figure 7B is a balance of force diagram of first and second mechanisms 116 and 117. In essence, first mechanism 116 creates a force $F_1$, for pressing tissue 44 against frame 110 of device 100, at proximal end 108, while second mechanism 117 imposes a force $F_2$, which is substantially opposite in direction to $F_1$, for pressing sensor 122 against tissue 44. Thus, tissue 44 is pressed against proximal end 108 by force $F_1$ while, simultaneously, sensor 122 is pressed against tissue 44 by force $F_2$.

Each mechanism provides a force opposite to that of the other mechanism. The force $F_1$ ensures that tissue 44 will not move away from sensor 122, under the force $F_2$, and the force $F_2$ ensures that sensor 122 will not move away from tissue 44, under the force $F_1$. Operating together, first and second mechanisms 116 and 117 ensure that effective sensor-to-tissue contact is maintained at interface 45.

Alternatively, first mechanism 116 imposes a force $F_1$, a component of which presses tissue 44 against frame 110 of device 100, at proximal end 108 thereof, while second mechanism 117 imposes a force $F_2$, a component of which presses sensor 122 against tissue 44, so that components of each mechanism provide a force opposite to that of the components of the other mechanism.

Thus, either the forces $F_1$ and $F_2$, or components thereof, ensure that tissue 44 will not move away from sensor 122 and that sensor 122 will not move away from tissue 44, so that effective sensor-to-tissue contact is maintained at interface 45.

Figure 7C schematically illustrates a longitudinal cross-section of the device of Figures 7A-B, for forming effective sensor-to-tissue contact,. The situation of Figure 7C is analogous to that of Figure 1I, hereinabove, but the male-female relationship is reversed. Whereas in Figure 1I, the element 20, on which the sensors 24 are situated, is operative as a female, receiving tissue 44, in Figure 7C, the sensor 122, which may also be referred to as the sensor 24, is operative as a male. It will be appreciated that Figure, 1N hereinabove, which illustrates effective contact is applicable to both situations.

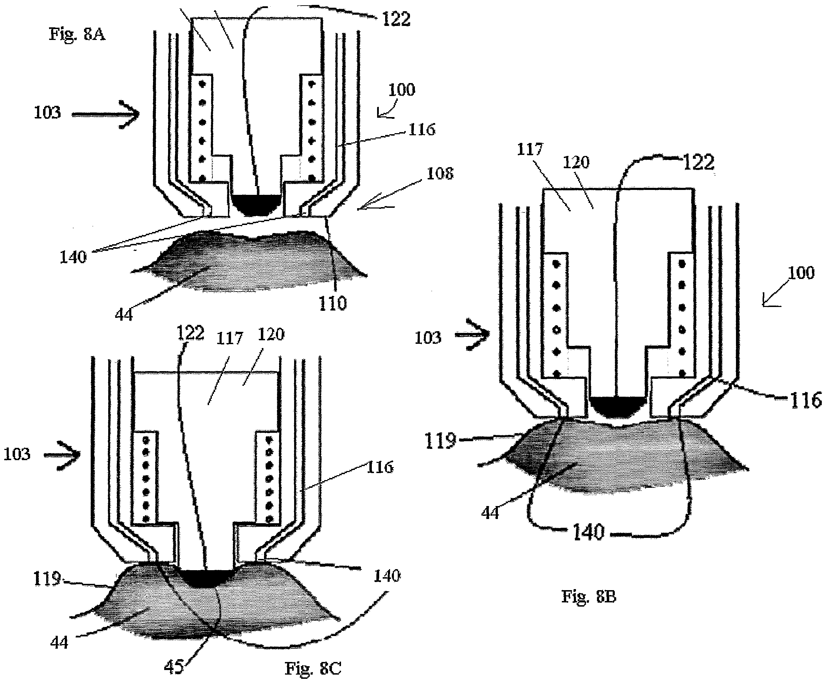

Reference is now made to Figures 8A-C, which schematically illustrate the operation of the device 100. As seen in Figure 8A, in a first step of operation, device 100, which is preferably a hand-held device, is physically brought proximal to tissue 44, for example, by hand. As seen in Figure 8B, in a second step, tissue 44 is attached by suction to device 100, via first mechanism 116. As seen in Figure 8C, in a third step, second mechanism 117, the piston 120, is deployed, for pressing sensor 122 against tissue surface 119, forming substantially the single interface 45. At this point, effective sensor-to-tissue contact has been formed, substantially, as shown in Figure 7B, and tissue characterization may take place via the sensor 122.

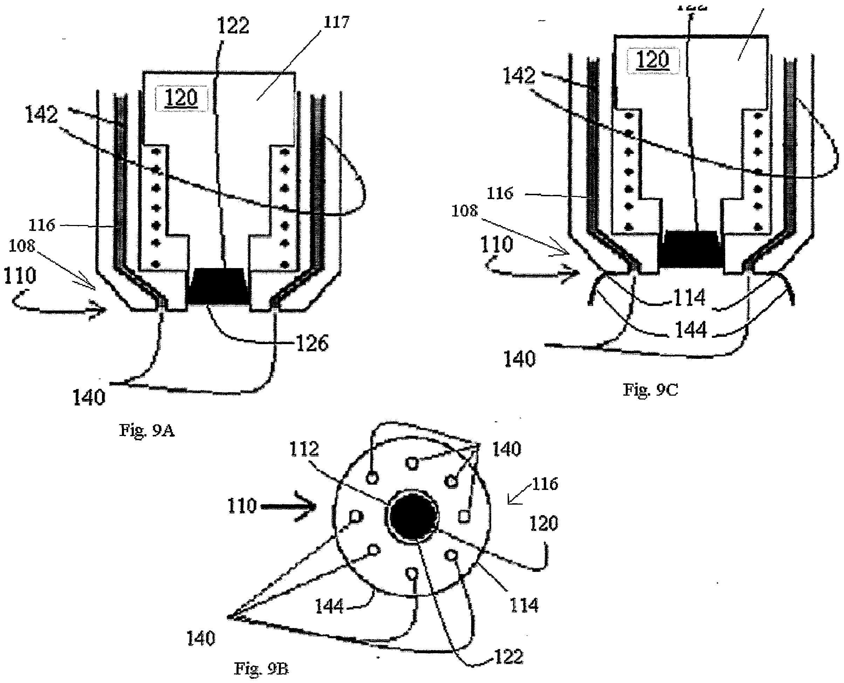

Reference is now made to Figures 9A-C, illustrating side and top cross-sectional views of a first mechanism 116 of a device 100. Frame 110 at proximal end 108 is preferably shaped as two concentric circles, having an inner diameter 112 and an outer diameter 114. Furthermore, inner diameter 112 is configured to allow piston 120 to advance therethrough, towards the surface 119 of tissue 44, so that proximal end 126 of piston 120 protrudes beyond frame 110, so as to press against the tissue which is attached to frame 110, as discussed above with reference to Figure 8C. Additionally, frame 110 is provided with at least two orifices 140, preferably arranged between circumferences 112 and 114. For example, eight orifices may be used, as shown in Figure 9B. Suction is employed, via duct system 142, through the orifices 140 for attaching tissue 44 to frame 110.

In Figure 9C a flexible skirt 144 is added to periphery of frame 110 for providing improved suction when attaching tissue 44 to proximal end 108. Preferably, flexible skirt 144 is made of a resilient material, for example, natural or synthetic rubber.

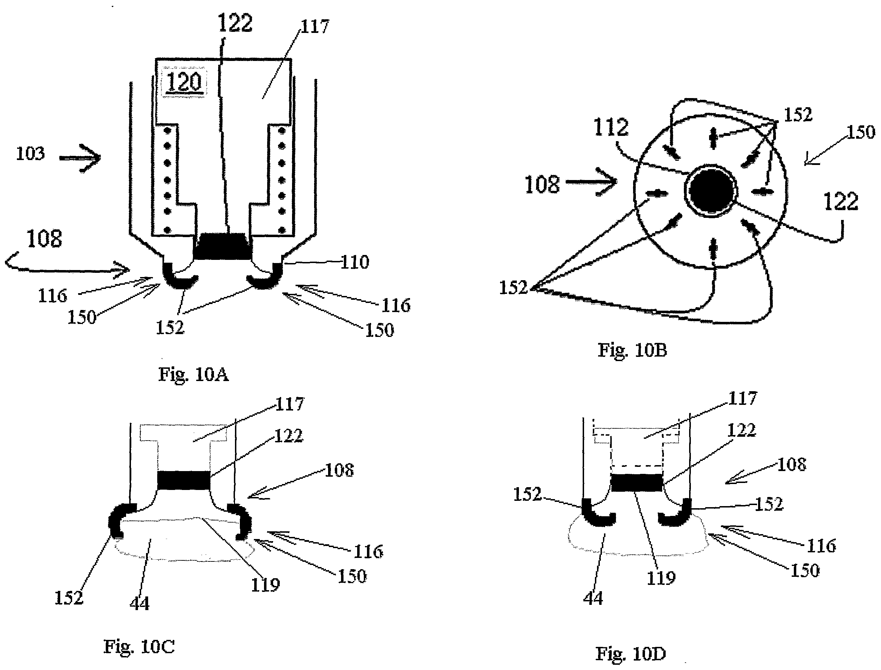

Reference is now made to Figures 10A-D, illustrating side and top cross-sectional views of the first mechanism 116 of a device employing a gripping device 150, as the first mechanism 116. Thus, the duct system 142 for creating suction need not be used.

According to the embodiment shown in Figures 10A-D, gripping device 150 is positioned on frame 110. Additionally, gripping device 150 comprises at least two claws 152 configured to be in either a retracted state (as shown in Figure 10C) or in a gripping state (as shown in Figure 10D). Preferably, the at least two claws 152 are arranged on frame 110 (eight claws are shown in Figure 10B). Prior to gripping tissue 44, proximal end 108 is positioned close to tissue surface 119 with at least two claws 152 being in a retracted state. The claws are then brought to a gripping state, thus attaching tissue 44 to proximal end 108.

At this point the sensor 122 may be brought down by the second mechanism 117, for creating effective sensor-to-tissue contact between sensor 122 of device 100 and tissue surface 119.

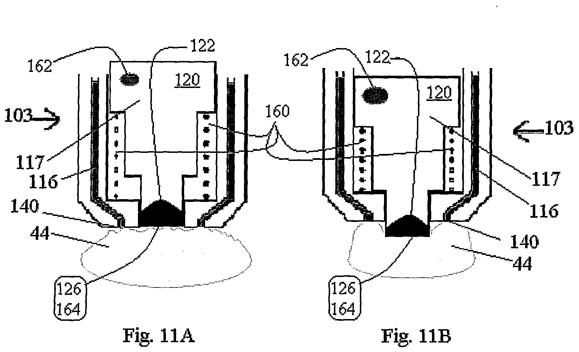

Reference is now made to Figures 11A-B, schematically illustrating a side cross-sectional view of the second mechanism 117, for forming effective sensor-to-tissue contact,. Second mechanism 117 preferably includes a piston 120 having a tissue characterizing sensor 122 at proximal end 126 thereof. Piston 120 is configured to slide smoothly from a retracted position (shown in Figure 11A) to a deployed position (shown in Figure 11B) in which effective contact between tissue 44 and tissue characterizing sensor 122 is established. Advancing piston 120 towards tissue 44 may be controlled by any known means, and will not be discussed here in detail. Movement of piston 120 towards tissue 44 is limited by at least one spring 160, which is compressed as the piston 120 moves toward the tissue 44. Furthermore, advancing piston 120 towards tissue 44 may be actuated using a switch 162. Preferably, a surface 164 of proximal end 126 of piston 120 is highly polished, for creating the effective contact with tissue 44.

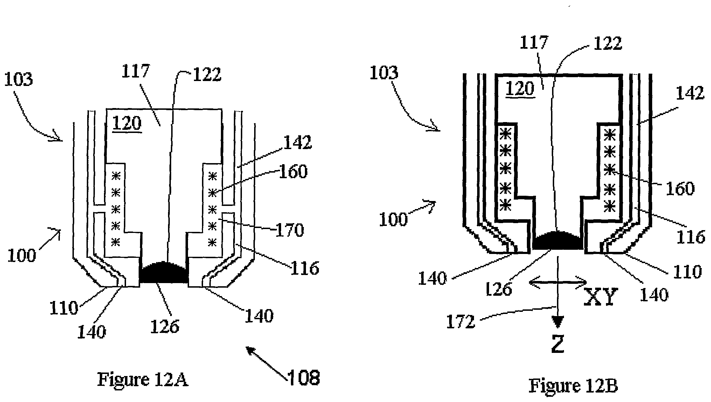

Reference is now made to Figure 12A, schematically illustrating a side cross-sectional view of second portion 103 of device 100 for tissue characterization. According to the present embodiment, spring 160 is contained within a spring tunnel 170, which is connected to duct system 142. Therefore, when orifices 140 are closed, piston 120 is actuated by the suction created in spring tunnel 170, responsive to the partial pressure in duct system 142, as first mechanism 116 attaches tissue 44 to device 100 at proximal end 108 (see Figure 11A-B).

Reference is now made to Figure 12B, schematically illustrating a side cross-sectional view of second portion 103 of device 100 for tissue characterization. In Figure 12B there is also illustrated a coordinate system 172. As piston 120 slides towards tissue (not shown) along a Z axis, perpendicular to frame 110, as discussed with reference to Figures 8B-C and 11A-B, it vibrates in an XY plane, perpendicular to the Z axis. The vibrational motion in the XY plane allows for a gliding motion against the tissue which, in effect, removes, as if by sweeping, any foreign matter, or entrapped gases or liquids, which may otherwise be present at the interface 45 (see Figures 1J and 1L).

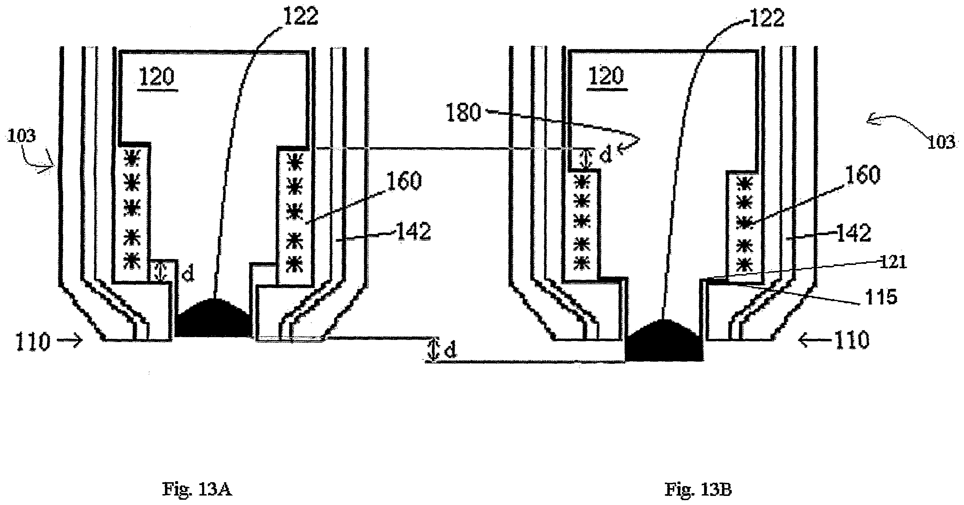

Reference is now made to Figures 13A-D, which schematically illustrate a means for controlling the travel of the piston 120. In Figures 13A and 13B, piston 120 is shown in retracted and deployed positions, respectively. It may be noted that, after the piston 120 has traveled distance d and has advanced to the position shown in Figure 13B, a portion 121 of piston 120 abuts a protruding portion 115 of second portion 103, thus halting movement of the piston 120. Thus, piston 120 has a fixed travel distance, d, shown at 180, wherein portion 115 of second portion 103 is configured to operate as a stop, to ensure that piston 120 does not travel more than the fixed travel distance d.

Alternatively, movement of the piston 120 need not be halted by the contact of a portion thereof with a protruding portion 115 of second portion 103, as discussed above. Instead, if desired, before piston portion 121 reaches protruding portion 115, movement of the piston may be halted by the action of a spring 160, configured such that, when the spring 160 has been compressed to its maximum value, the desired fixed travel distance d has been reached. Preferably, distance d is between about 1mm and about 50mm.

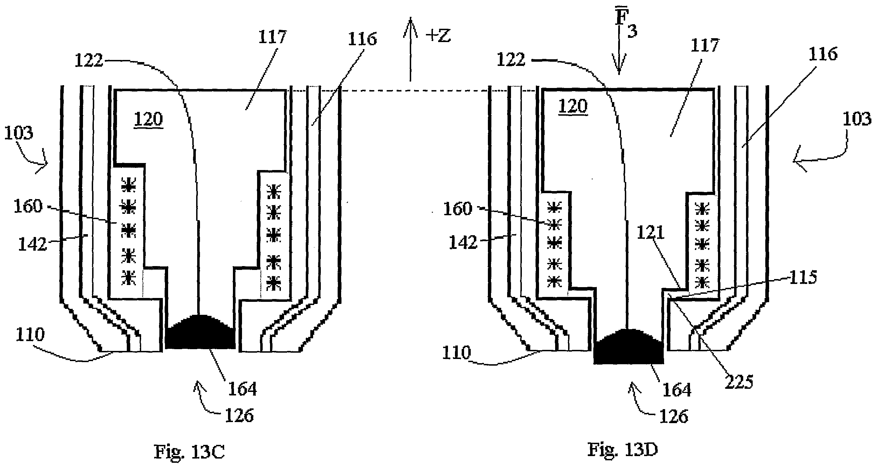

Figures 13C and 13D schematically illustrate piston 120 in retracted and deployed positions, respectively. The advancement of piston 120 and of sensor 122 is such that a force $F_3$, exerted on piston 120, is exactly balanced by the force which the attached tissue (not shown) imparts to sensor 122, in the +Z direction, and by the force compressed spring 160 imparts to piston 120 in the +Z direction. It should be noted that, unlike the embodiment shown in Figures 13A-B, in this embodiment after the piston 120 has advanced to the position shown in Figure 13D, there remains a certain amount of clearance 225 between portion 121 of piston 120 and protruding portion 115 of second portion 103.

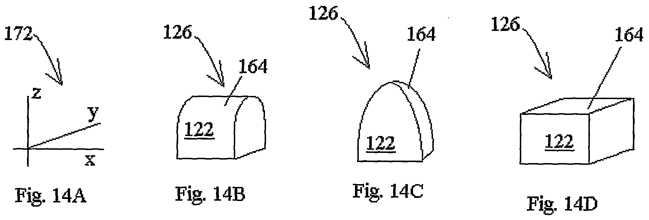

Reference is now made to Figures 14A-D, which schematically illustrate a coordinate system and various geometries for the proximal end 126 of the sensor 122,. Figure 14A provides a reference coordinate system 172. Figures 14B-D schematically illustrate a roller-shaped sensor 122, a dome-shaped sensor 122, and a cube-shaped sensor 122, respectively. Each sensor 122 is provided with a polished surface 164, at the proximal end 126 thereof. It will be appreciated that other shapes are also possible for the proximal end of the sensor.

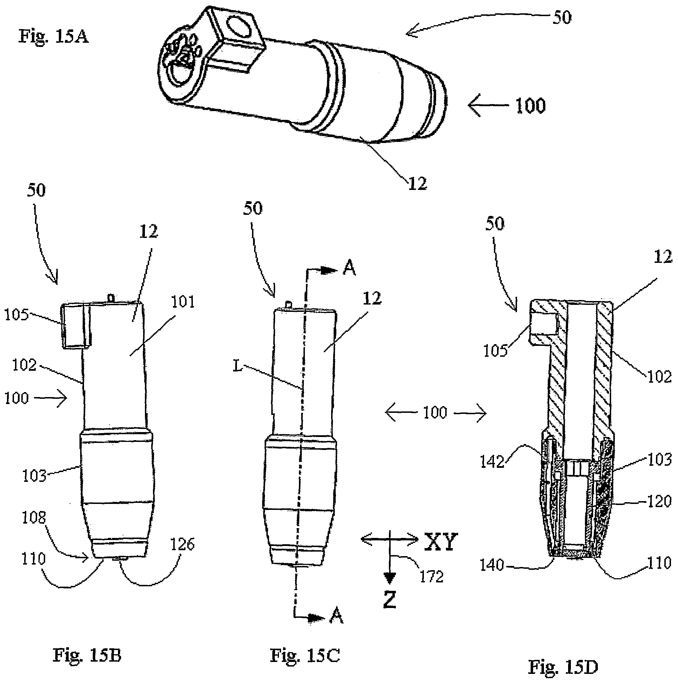

Reference is now made to Figures 15A-D, schematically illustrating a prototype of the probe 50, with the device 100 for tissue characterization, in. The device 100 is enclosed in the housing 12 of the probe 50.

Figures 15A and 15B are perspective and side views of device 100, respectively. Figure 15C is another side view of device 100, taken by rotating the device of Figure 15B 90 degrees about the Z axis, and Figure 15D is a cross-sectional view, taken in the direction of arrows A-A of Figure 15C. Prototype device 100, as illustrated, is generally operated when perpendicular to a tissue, for example, as shown in Figure 8A, so that its length L is parallel to the Z axis, while tissue surface 119 (Figs. 8A-8C) is generally parallel to the X-Y plane. Device 100 preferably has a length of between about 50mm and about 300mm, and a diameter of between about 10mm and about 150mm. It will be appreciated that, if desired, other dimensions are possible for device 100. Structure 101 of device 100 is preferably made of a rigid material, for example, a rigid plastic, ceramic, wood, or the like. Vacuum outlet 105 is adapted for connecting to a pump (not shown).

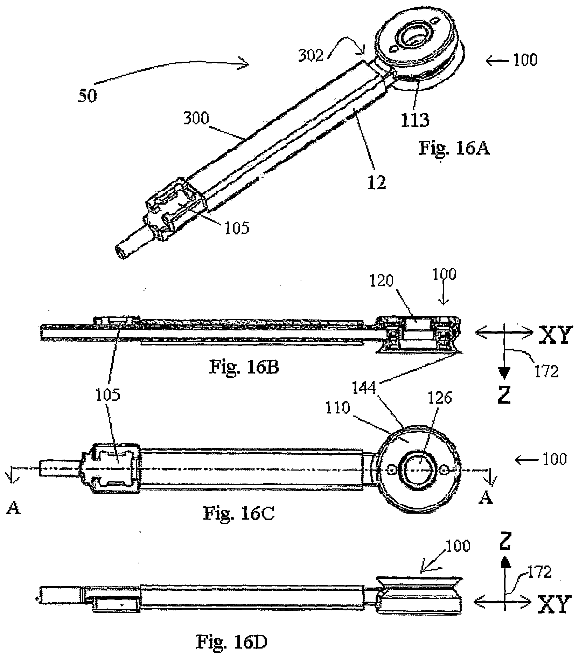

Reference is now made to Figures 16A-D, which schematically illustrate the probe 50, with the device 100 for tissue characterization. The device 100 is enclosed in the housing 12 of the probe 50.

Figures 16A and 16D are perspective and side views of probe 50 and the device 100, respectively. Figure 16C is another side view of probe 50 and the device 100, taken by rotating the device of Figure 16D 90 degrees about the Z axis, and Figure 16B is a cross-sectional view, taken in the direction of arrows A-A of Figure 16C. The probe 50, as illustrated, is shaped as an arm 300 having a ring construction 113 at a proximal end 302 thereof. Prototype device 100 illustrated in Figures 16A-D is generally operated when arm 300 is held parallel to a tissue (not shown), so that the tissue surface is generally parallel to the X-Y plane and the sensor in second mechanism 117 moves in a direction generally parallel to the Z axis. Preferably, a flexible skirt 144 is provided at a proximal end of ring construction 113 for assisting in attaching the tissue to frame 110. It will be appreciated that the device illustrated in Figures 16A-D may include a first mechanism and/or a second mechanism similar to any of those discussed with regard to Figures 7A-13D above.

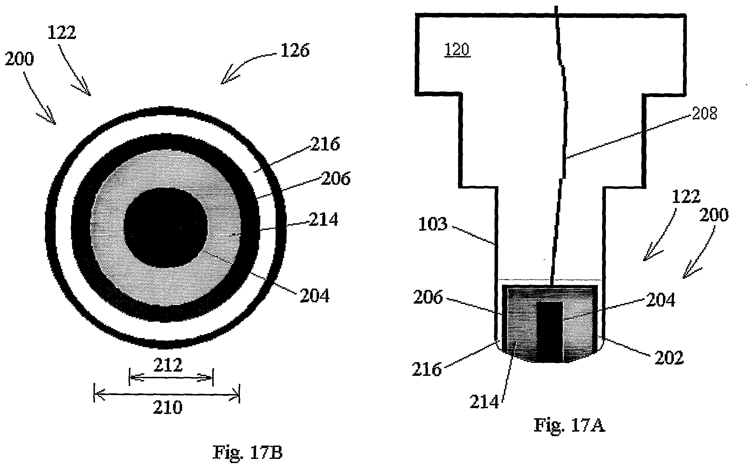

Reference is now made to Figures 17A-E, schematically illustrating cross-sectional views of a piston 120 containing a sensor 122. The illustrations show a variety of sensors for characterizing the tissue after the tissue surface is brought into effective contact with the sensor.

Sensor 122 may be a dielectric-property sensor, an optical sensor, an MRI sensor, an RF sensor, an MW sensor, an X-ray sensor, an ultrasound sensor, a biosensor, a chemical sensor, a mechanical sensor, a temperature sensor, an infrared thermography sensor, or any other sensor suitable for characterizing tissue.

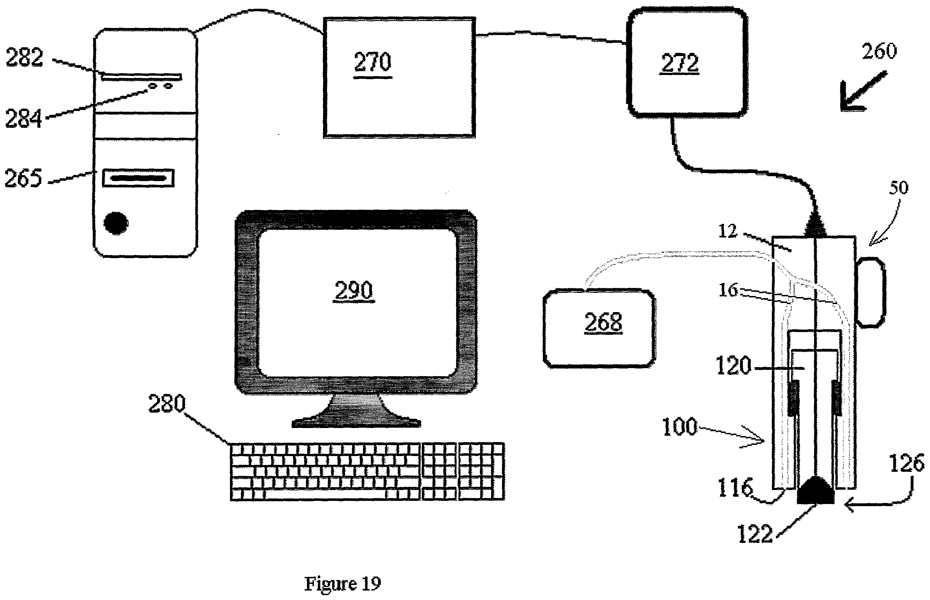

Figures 17A and 17B illustrate a side cross-sectional view and a view from proximal end 126, respectively, of piston 120 containing sensor 122. According to the present embodiment, sensor 122 is a dielectric-property sensor 200, for measuring the dielectric properties of tissue, for example, as taught by commonly owned US Patent 6,813,515 and commonly owned PCT Publication WO 03060462A. By comparing the results with known dielectric properties of tissues, or by evaluating generated pulses with those reflected from the tissue, the characteristics of the tissue can be determined. Furthermore, dielectric-property sensor 200 may be constructed as a coaxial cable 202, having an inner electrode 204 and an outer electrode 206 separated by an insulating sheath 214 made of, for example, Teflon©. Outer electrode 206 may be grounded. Preferably, coaxial cable 202 is located within piston 120. Signals from dielectric-property sensor 200 are transferred for analysis through a transmission line 208 to a computerized system 260, described hereinbelow in conjunction with Figure 19.

Preferably, inner electrode 204 has a diameter 212 of between about 0.2 and 1.5 mm and the inner surface of outer electrode 206 has a diameter 210 of between about 3.0 and 10.0 mm. Outer electrode 206 is about 0.5 mm thick. It will be appreciated that other dimensions, which may be larger or smaller, may similarly be used. The dielectric-property sensor 200 may be encased in a filler material 216, for example epoxy, which may be formed as a plug that fits into piston 120.

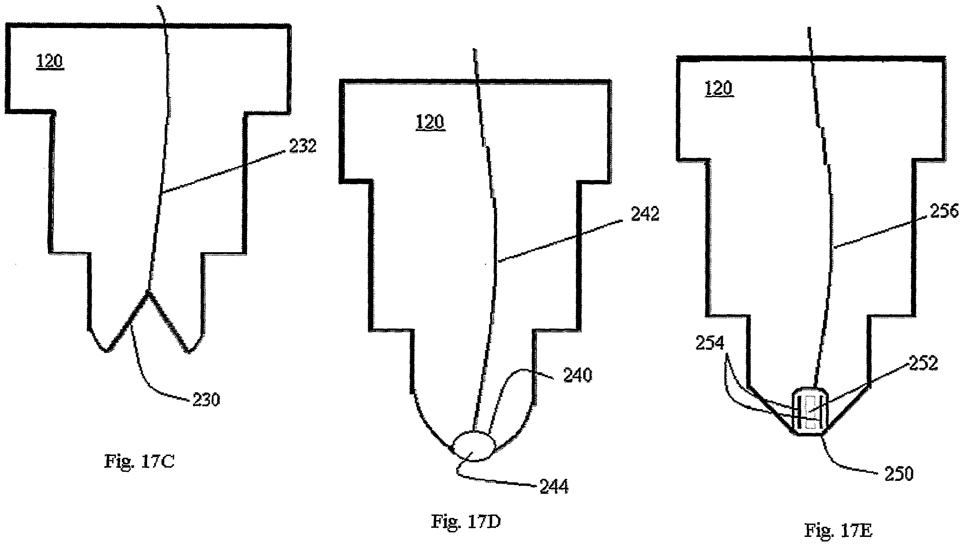

FIG. 17C illustrates a piston 120 wherein the sensor is formed as an RF or MW horn antenna 230 mounted on piston 120.

RF or MW horn antenna 230 is associated with an RF/MW transmission line or wave guide 232, while units 272 and 270 (FIG. 19) are RF/MW generation and analysis units, respectively. The present embodiment relies on RF microwave characterization by the generation of propagating radiation in the RF microwave region of the electromagnetic spectrum, towards the tissue, and the measuring of its reflection. The radiation is usually transmitted and received by an antenna, for example the horn antenna 230. The tissue characterization is done by analyzing the amplitude and phase difference between the original waves and the reflected waves.

Figure 17D illustrates piston 120 wherein the sensor is formed as an optical sensor 240 mounted on piston 120. An optical signal is generated in an external unit, such as unit 272 (FIG. 19), and is transmitted via an optical fiber 242 to the tissue. The reflection of the light is then received in a dedicated module inside the optical unit. Preferably, the optical energy is transmitted to and from tissue 44 via a lens 244.

The details of optical signal generation, reception and analysis depend on the specific optical method that is chosen. For example, for reflection spectroscopy, tissue characterization relies on measuring the relative amplitude and phase of the reflected light versus the generated light. An example of the reflection spectroscopy method is described in commonly owned US Patent Application 10/298196. It will be appreciated that any other suitable method may be used.

Alternatively, auto florescence may be used for measuring radiation emitted from the tissue at a wavelength different from that originally transmitted. The emitted radiation occurs in response to excitation by impinging radiation, and may be used for tissue characterization, for example, as used by Xillix Technologies Corp., and as described in http://www.xillix.com/index_home.cfm. It will be appreciated that any other suitable method may be used.

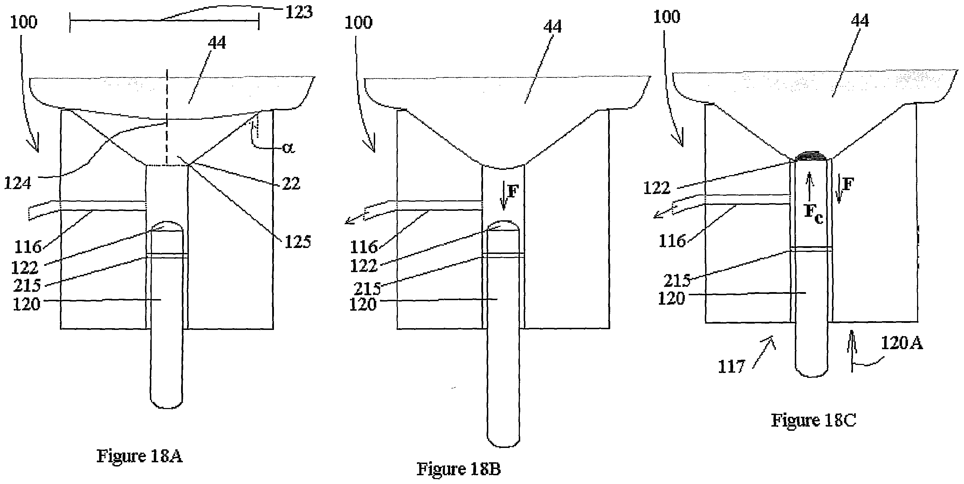

FIG. 17E illustrates a piston 120 having an MRI sensor 250 mounted thereon. MRI sensor 250 has a permanent magnet 252, enclosed in an RF coil 254, for example, as taught in commonly owned US Patent Application 2005/0021019 to Hashimshony, et al., entitled "Method and Apparatus for Examining Substance, Particularly Tissue, to Characterize its Type," and in U.S. Patent 5,572,132 to Pulyer, et al., entitled "MRI Probe for External Imaging,". Referring further to the drawings, Figures 18A-C schematically illustrate another configuration for effective contact, employing a three step procedure in the operation of the device 100. In accordance with the present embodiment, the device 100 is provided with a sensor 122 located on the piston 120, but no sensors are provided on the rigid surface 22. It may be noted that the present embodiment employs a mechanism incorporating a portion having a rigid surface 22 for providing effective contact which is somewhat similar to that discussed above with regard to, for example, Figure 2A, as well as a mechanism similar to the second mechanism 117 shown in, for example, Figure 7A.

As seen in Figure 18A, in a first step of operation, device 100, is physically brought proximal to tissue 44, for example, by hand. As seen in Figure 18B, in a second step, tissue 44 is attached by suction to device 100, via first mechanism 116. As seen in Figure 18C, in a third step, a second mechanism 117 is deployed, for pressing the sensor 122 of piston 120 against tissue surface 119, thus forming an effective sensor-to-tissue contact, whereat tissue characterization may take place via the sensor 122.

Again, the line of force F is at a sharp angle $\alpha$ to the rigid surface 22 of the conical structure 125, fixing the tissue 44 to the rigid surface 22.

As a third step, seen in Figure 18C, the counter mechanism, for the counter force $F_c$ in opposition to the force F, is provided by the piston 120, moving in a direction of an arrow 120A, and pressing the at least one piston sensor 122 against the immobilized tissue 44, achieving effective contact between the piston sensor 122 and the portion of the immobilized tissue 44 in contact with the piston sensor 122.

Reference is now made to Figure 19, schematically illustrating an overall system 260 for tissue characterization, in accordance with an embodiment of the present invention.

Preferably, the device 100 is housed in the housing 12, and includes the communication architecture 16 to form the probe 50. The probe 50 may be extracorporeal and hand-held, or adapted for intracorporeal insertion, as described further hereinbelow.

System 260 includes the probe 50, having the device 100 with the at least one sensor 122. A signal generating unit 272 provides sensor 122 with a signal and a signal analyzing unit 270 receives and analyzes signals from sensor 122. It will be appreciated that signal generating unit 272 and signal analyzing unit 270 may be integrated into a single unit. For example, where the sensor 122 is a dielectric-property sensor constructed essentially as a coaxial cable, units 270 and 272 may include, for example, an impedance analyzing external unit, such as Hewlett-Packard's Agilent 4396A, and a test fixture connected via a coaxial cable to the impedance analyzing external unit. The sensors may be battery operated or associated with power supply units.

It will be appreciated that units 270 and 272 will correspond to the specific sensor 122 employed in device 100. For example, where sensor 122 is an optical sensor, unit 272 may be a light emitting diode or a laser, and unit 270 may be an optical analyzer or a CCD.

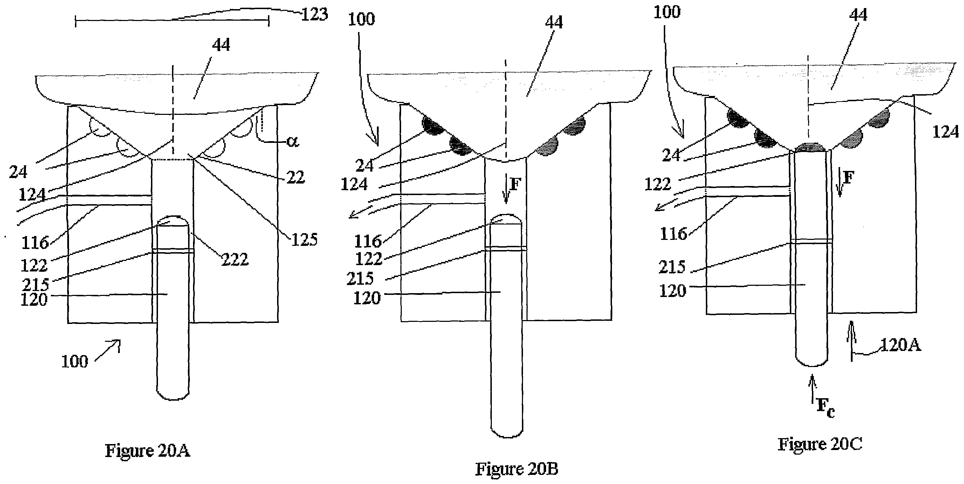

A control and processing unit, such as a computer 265, which may be a personal computer, a laptop, a palmtop, a microcomputer, or any other suitable computer, may also be used for additional data analysis. Preferably, control and processing unit 265 includes a user interface 280, for example, a keyboard or knobs, and may further include a storage system, such as a read and write drive 282, a USB port 284, and a display screen 290. Referring further to the drawings, Figures 20A-C schematically illustrate a device 100 for effective contact, in accordance with another embodiment of the present invention. As described here, however, the device 100 has a substantially conical construction 125, configured for making contact with a tissue.

It will be appreciated that the control and processing unit 265 may be integrated with the signal generating unit 272 and signal analyzing unit 270.

It will be appreciated that, alternatively, if desired, the structure 125 may have any suitable configuration. For example, structure 125 may be elliptical or circular in cross section, as described above with reference to Figures (1O and 1S) and (1P and 1T), respectively.

As illustrated in Figures 20A-C, the substantially conical construction 125 defines a circle having an effective diameter 123 and a longitudinal axis 124. The device 100 operates by two mechanisms, similar to those discussed above with reference to Figures 7A-C. A first mechanism 116, which exerts a force F on the tissue 44 along the longitudinal axis 124, fixes the tissue 44 against the device 100, so as to substantially immobilize the tissue 44. A second mechanism 117, which presses a piston sensor 122 associated with device 100 against the immobilized tissue 44, exerts a counter force $F_c$ in opposition to at least a component of the force F, thus achieving effective contact between the surface 44 and the piston sensor 122. These mechanisms have been discussed above, for example, with regard to Figures 8A-C and will not be discussed here in further detail.

As shown in Figures 20A-C, seals 215 may be provided between the piston 120 and the inner walls 222 of the device 100 in order to ensure the integrity of the vacuum in the vacuum line 116.

It will be further appreciated that one or several sensors 24 may be mounted on the rigid surface 22, arranged so as to provide three dimensional information of the tissue 44. Thus, the sensors 24 will operate at the interface of the tissue 44 with the rigid surface 22 which provides effective contact formed by the force F at the sharp angle $\alpha$ to the rigid surface 22 (Figure 20A), for stretching or stretching and pushing the tissue 44 against the rigid surface 22. Operation of the sensors 24 will not, however, be affected by the second mechanism 117, whereby the at least one piston sensor 122 is pressed against the tissue 44, with the force $F_C$, seen in Figure 20C.

As noted above, each of the at least one piston sensor 122 and the at least one sensor 24 may be an optical sensor, an X-ray sensor, an RF sensor, an MW sensor, an infrared thermography sensor, an ultrasound sensor, an MR sensor, an impedance sensor, a temperature sensor, a biosensor, a chemical sensor, a radioactive-emission sensor, a mechanical sensor, a nonirradiative RF sensor, or any other sensor suitable for tissue characterization.

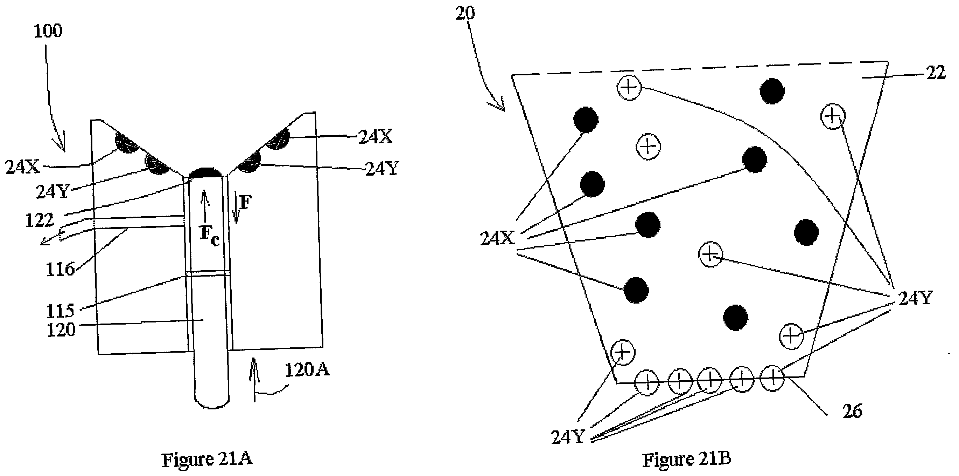

It will be appreciated that the device shown in Figures 20A-C may operate in conjunction with a system similar to that discussed above with reference to Figure 19. However, such a system may include a signal generating unit and a signal analyzing unit (or a combined signal generating and analyzing unit) for the sensors of each of the first and second mechanisms 116 and 117. Figures 21A-B schematically illustrate configurations with several types of sensors, in accordance with embodiments of the present invention.

As seen in Figure 21A, three types of sensors may be used, sensors 24X and 24Y along the conical construction 125, and a piston sensor 122 on the piston 120. This arrangement will provide three-dimensional information by combining information received by both the sensor types 24X and 24Y. In this manner, three-dimensional information for example, by ultrasound and optical sensors, or by MRI and X-ray may be obtained and compared. It will be appreciated that many combinations of different sensor types are possible.

It will be noted that, in the embodiment shown in Figure 21A, the piston is provided with a single sensor 122 and, as such, is not configured so as to provide three-dimensional information. Alternatively, if desired, the piston may be provided with a plurality of sensors of different types, so as to provide three-dimensional information, in a manner similar to that of the sensors 24X and 24Y of conical construction 125, discussed below.

As seen in Figure 21B, two types of sensors 24X and 24Y are provided, randomly positioned on the rigid surface 22. This arrangement will provide three-dimensional information regarding the tissue. Again, three-dimensional information of different modalities may be obtained and compared.

The sensors 24X, 24Y, and 122 may be, for example, irradiative sensors, such as optical sensors, X-ray sensors, RF sensors, MW sensors, infrared thermography sensors, and ultrasound sensors; nonirradiative sensors, such as MR sensors, impedance sensors, temperature sensors, biosensors, chemical sensors, radioactive-emission sensors, mechanical sensors, and nonirradiative RF sensors; or any other suitable sensor, or any combination thereof.

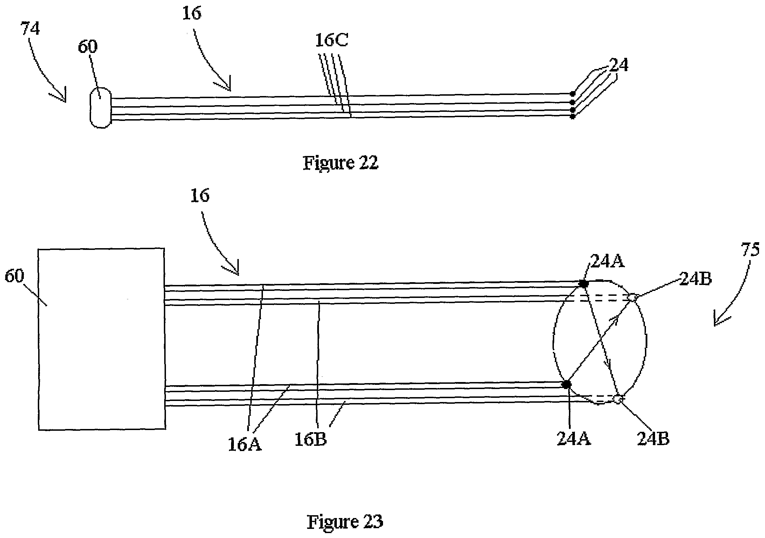

Figure 22 schematically illustrates a first sensor construction 74 for the device 10, in accordance with some embodiments of the present invention. The first sensor construction 74 is applicable to sensors 24, wherein each is operative as both a transmitter and a receiver. Alternatively, the first sensor construction 74 is applicable to sensors 24, operative as receivers of natural signals, for example, body temperature sensors, where no transmission is necessary. Accordingly, the first sensor construction 74 includes the signal generating and analyzing unit 60 (Figure 3), the signal communication architecture 16, of signal communication lines 16C, communicating with each sensor, and the sensors 24, each operative as a transmitter and a receiver.

Figure 23 schematically illustrates a second sensor construction 75, for the device 10, where a sensor 24A is a transmitter and a sensor 24B is a receiver, in accordance with some embodiments of the present invention. Accordingly, the second sensor construction 75 includes the signal generating and analyzing unit60. The signal communication architecture 16 includes a signal communication line 16A to each transmitting sensor 24A, and a receiving line 16B from each receiving sensor 24B.

Figures 24A and 24B schematically illustrate optical sensor constructions for the device 10, in accordance with some embodiments of the present invention.

In accordance with one embodiment, seen in Figure 24A, an optical sensor construction 76 includes optical signal generators 60A, such as lasers or LEDs, and optical signal analyzers 60B, formed, for example, as CCDs. The signal communication architecture 16 includes optical fibers 16A, leading from the optical signal generators 60A to the transmitting sensors 24A at the tissue, and optical fibers 16B, leading from the receiving sensors 24B at the tissue to the optical signal analyzers 60B. The sensors 24A and 24B are at the proximal endings of the respective optical fibers 16A and 16B, relative to the tissue.

In accordance with another embodiment, seen in Figure 24B, an optical sensor construction 78 includes the optical signal generators 60A, such as the lasers or the LEDs, and the optical signal analyzers 60B are, for example, formed as the CCDs. The signal communication architecture 16 includes optical fibers 16C, leading both from the optical signal generators 60A to the transmitting sensors 24A at the tissue and from the receiving sensors 24B at the tissue to the optical signal analyzers 60B. Beam splitters 60C, at the distal end with respect to the tissue, direct the beam from the optical signal generators 60A to the optical fibers 16 and from the optical fibers 16 to the optical signal analyzers 60B. The sensors 24A and 24B are at the proximal endings of the optical fibers 16, with respect to the tissue. Other techniques for using a single optical fiber both for transmitting and receiving optical signals may also be used. It will be appreciated that, if desired, different types of sensors may be used for transmitting sensors 24A and for receiving sensors 24B. Additionally, it will be appreciated that any other suitable signal communication architecture may be used.

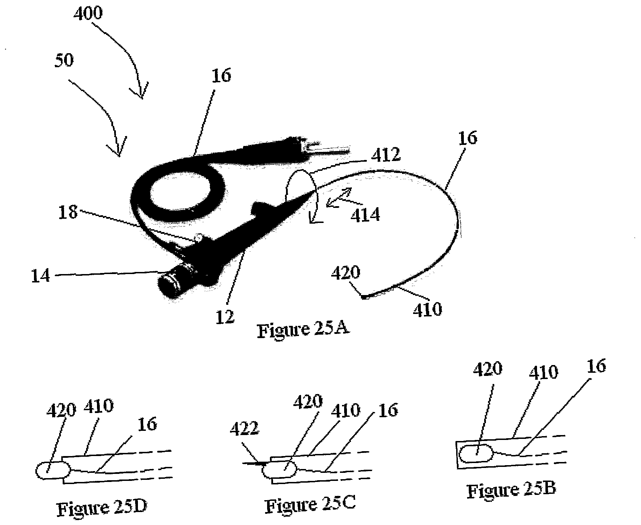

Referring further to the drawings, Figures 25A - 25D schematically illustrate the probe 50 as an endoscopic probe 400, in accordance with embodiments of the present invention.

The endoscopic probe 400 may be adapted for endoscopic insertion via a body orifice or percutaneously.

Preferably, the endoscopic probe 400 includes the housing 12, adapted for endoscopic insertion, the gripping handle 14, the at least one control switch 18, and the communication architecture 16, a display 135 (Figure 2A) may also be provided near the control switch 18.

The housing 12 includes an endoscopic cable 410, which is preferably configured for lateral motion along an arrow 412 and rotational motion along an arrow 414.

The endoscopic probe 400 further includes a device 420 for tissue characterization, constructed as any one of the embodiments taught hereinabove, in conjunction with Figures 1A - 24B.

The device 420 may be retracted within the cable 410, during insertion and other maneuvering, and deployed for characterization.

Additionally, the device 420 or the probe 400 may include a sharp edge 422, which may be retracted unless penetration through tissue is required, for example, through the skin, through a body lumen or through another tissue. The sharp edge may be associated with the device 420 or with the cable 410.

Figure 25A schematically illustrates the overall endoscopic probe 400.

Figure 25B schematically illustrates the device 420, retracted in the cable 410, for insertion or other maneuvering.

Figure 25C schematically illustrates the device 420 with the sharp edge 422 deployed, for penetrating some tissue.

Figure 25D schematically illustrates the device 420, deployed for tissue characterization.

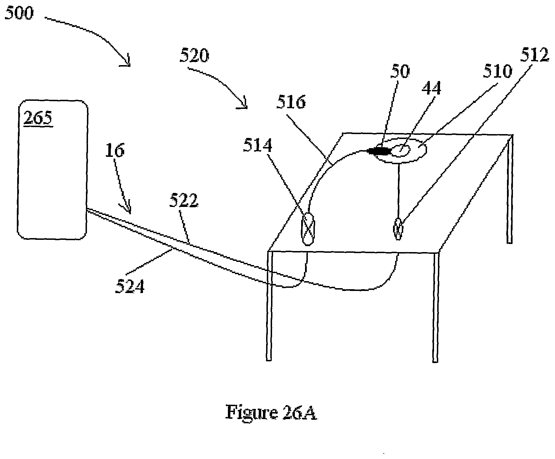

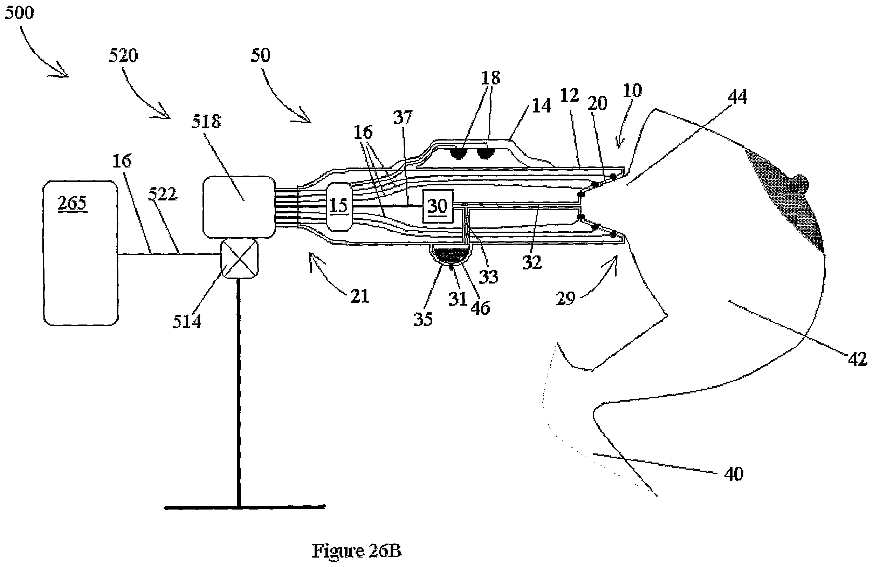

Referring further to the drawings, Figures 26A and 26B schematically illustrate the probe 50 as an extracorporeal probe, in an examination station 500, in accordance with embodiments of the present invention.

As seen in Figure 26A, the examination station 500 may be adapted for ex-vivo tissue or biopsy samples, and may include a gantry 520, having a motion provider 514 and an arm 516, to which the probe 50 is attached.

The tissue 44 may be positioned on another gantry 510, having another motion provider 512.

The gantries 520 and 510 may be automatically controlled, for example, by the control and processing unit 265, via communication lines 522 and (or) 524, which may also operate as the signal communication architecture 16.

The gantries include at least one degree of freedom, but preferably as many as 6 degrees of freedom for rotational and translational motions.

Alternatively, only one motion provider, either 514 or 512 may be used.

It will be appreciated that the motion may be manually controlled.

Alternatively, the probe 50 is fixed in place, for example, attached to a fixed arm 516, while the tissue may be manipulated manually.

The control and processing unit 265 may also include the signal analysis and the signal generation units.

As seen in Figure 26B, the examination station 500 may be adapted for tissue in situ, and may include the gantry 520, having the motion provider 514 and a coupler 518, to which the probe 50 is attached.

The gantry 520 may be automatically or manually controlled.

The control and processing unit 265, which may also include the signal analysis and the signal generation units, may control the operation of the gantry 520.

The gantry 520 includes at least one degree of freedom, but preferably as many as 6 degrees of freedom for rotational and translational motions.

The following relates to a probe for substance characterization, constructed by the teachings of any one of the embodiments taught hereinabove, in conjunction with Figures 1A-26B.

In accordance with some embodiments of the present invention, the probe may be a hand-held probe, and may include a gripping handle.

In accordance with some embodiments of the present invention, the probe may be mounted on a gantry, for providing it with automated or controlled motion.

In accordance with some embodiments of the present invention, the probe may be fixed to an examination station.

In accordance with some embodiments of the present invention, the probe may be adapted for characterizing a portion of ex-vivo tissue.

In accordance with some embodiments of the present invention, the probe may be adapted for characterizing a biopsy sample.

In accordance with some embodiments of the present invention, the probe may be adapted for characterizing a portion of tissue in situ.

In accordance with some embodiments of the present invention, the probe may be adapted for characterizing a portion of skin.

In accordance with some embodiments of the present invention, the probe may be adapted for characterizing a portion of subcutaneous tissue.

In accordance with some embodiments of the present invention, the probe may be adapted for the characterizing of the portion of subcutaneous tissue, in open surgery.

In accordance with some embodiments of the present invention, the probe may be employed for minimally invasive surgery, for example, for insertion via a trocar valve, or for another percutaneous insertion, for the characterizing of the portion of subcutaneous tissue.

In accordance with some embodiments of the present invention, the probe may be an intracorporeal probe, adapted for insertion via body orifice to a body lumen, for characterizing a portion of inner lumen wall.

In accordance with some embodiments of the present invention, the probe may be an intracorporeal probe, adapted for percutaneous insertion to a body lumen, for characterizing a portion of inner lumen wall.

In accordance with some embodiments of the present invention, the probe may be an intracorporeal probe, adapted for insertion via body orifice to a body lumen, and for penetrating the lumen, for characterizing a portion of subcutaneous tissue.

In accordance with some embodiments of the present invention, the probe may be an intracorporeal probe, adapted for percutaneous insertion to a body lumen, and for penetrating the lumen, for characterizing a portion of subcutaneous tissue.

In accordance with some embodiments of the present invention, the probe may be adapted for characterizing substance of non-biological tissue.

In accordance with some embodiments of the present invention, the probe and the system for tissue characterization may employ various types of sensors and techniques, including any one from the following nonexhaustive list.

Tissue characterization by ultrasonography: Ultrasonography is a medical imaging technique, using high frequency sound waves in the range of about 1 to 40 MHz and their echoes. The sound waves travel in the body and are reflected by interfaces between different types of tissues, such as between a healthy tissue and a denser, cancerous tissue, or between a portion of a soft tissue and a bone. The ultrasound probe receives the reflected sound waves and the associated instrumentation calculates the distances from the probe to the reflecting boundaries.

The ultrasound probe includes a piezoelectric crystal, which produces an electric signal in response to a pressure pulse. The shape of the probe determines its field of view, and the frequency of the emitted sound determines the minimal detectable object size. Generally, the probes are designed to move across the surface of the body. However, some probes are designed to be inserted through body lumens, such as the vagina or the rectum, so as to get closer to the organ being examined.

Before the early 1970's ultrasound imaging systems were able to record only the strong echoes arising from the outlines of an organ, but not the low-level echoes of the internal structure. In 1972 a refined imaging mode was introduced called grayscale display, in which the internal texture of many organs became visible. In consequence, ultrasound imaging became a useful tool for imaging tumors, for example, in the liver.

A development of recent years is 3D ultrasound imaging, in which several two-dimensional images are acquired by moving the probes across the body surface or by rotating probes, inserted into body lumens. The two-dimensional scans are then combined by specialized computer software to form 3D images.

In multiple-element probes, each element has a dedicated electric circuit, so that the beam can be "steered" by changing the timing in which each element sends out a pulse. By sequentially stimulating each element, the beams can be rapidly steered from left to right, to produce a two-dimensional cross-sectional image. Additionally, transducer-pulse controls allow the operator to set and change the frequency and duration of the ultrasound pulses, as well as the scan mode of the machine. A probe formed of array transducers has the ability to be steered as well as focused.

Contrast agents may be used in conjunction with ultrasound imaging, for example as taught by U.S. Patent 6,280,704 to Schutt, et al., entitled "Ultrasonic Imaging System Utilizing a Long-Persistence Contrast Agent,".

Tissue characterization by its dielectric properties: There are several known techniques for local tissue characterization by the tissue's electromagnetic properties.

Commonly owned US Patent 6,813,515 to Hashimshony, entitled "Method and System for Examining Tissue According to the Dielectric Properties Thereof," describes a method and system for examining tissue in order to differentiate it from other tissue, according to the dielectric properties of the examined tissue. The method includes applying an electrical pulse to the tissue to be examined via a probe formed with an open cavity such that the probe generates an electrical fringe field in examined tissue within the cavity and produces a reflected electrical pulse therefrom with negligible radiation penetrating into other tissues or biological bodies near the examined tissue; detecting the reflected electrical pulse; and comparing electrical characteristics of the reflected electrical pulse with respect to the applied electrical pulse to provide an indication of the dielectric properties of the examined tissue.

Furthermore, commonly owned US Patent Application 60/641,081 entitled "Device and Method for Tissue Characterization in a Body Lumen, by an Endoscopic Electromagnetic Probe," discloses a device and method for tissue characterization in a body lumen, for the detection of abnormalities, using an electromagnetic probe mounted on an endoscope. The endoscope may be designed for insertion in a body lumen, selected from the group consisting of an oral cavity, a gastrointestinal tract, a rectum, a colon, bronchi, a vagina, a cervix, a urinary tract, and blood vessels. Additionally, it may be designed for insertion in a trocar valve.

Additionally, commonly owned US Patent Application 60/665,842 entitled "Electromagnetic Sensors for Tissue Characterization," discloses a sensor comprising: a resonating element, formed as a conductive structure, configured to be placed proximally to an edge of a tissue for characterization, without penetrating the tissue. The resonating element has a diameter-equivalent, D, which defines a cross-sectional area thereof, on a plane substantially parallel with the edge, and at least one conductive lead, for providing communication with an external system, wherein the resonating element is configured to resonate at a free-air wavelength range of between about $\lambda$ and about $10\lambda$, wherein $\lambda$ is at least about ten times the diameter-equivalent D. Upon receiving a signal in the range of between about $\lambda$ and about $10\lambda$, the sensor is configured to induce electric and magnetic fields, in a near zone, in the tissue, the near zone being a hemisphere having a diameter of substantially D, beginning with the edge, while causing negligible radiation in a far zone, so that the tissue, in the near zone, effectively functions as part of the resonating element, varying a resonating response to the sensor, and so the tissue, in the near zone, is thereby characterized by its electromagnetic properties, by the resonating response to the sensor.

Tissue characterization by electrical impedance imaging: Electrical impedance imaging relates to measuring the impedance between a point on the surface of the skin and some reference point on the body of a patient. Sometimes, a multi-element probe, formed as a sheet having an array of electrical contacts, is used for obtaining a two-dimensional impedance map of the tissue, for example, the breast. The two-dimensional impedance map may be used, possibly in conjunction with other data, such as mammography, for the detection of cancer.

The article by Rajshekhar, V., entitled "Continuous Impedance Monitoring During CT-Guided Stereotactic Surgery: Relative Value in Cystic and Solid Lesions," in the British Journal of Neurosurgery, 1992, 6, pp. 439-444, describes using an impedance probe with a single electrode to measure the impedance characteristics of lesions. The objective of the study was to use the measurements made in the lesions to determine the extent of the lesions and to localize the lesions more accurately. The probe was guided to the tumor by CT and four measurements were made within the lesion as the probe passed through the lesion. A biopsy of the lesion was performed using the outer sheath of the probe as a guide to position, after the probe itself was withdrawn.

U.S. Patent 4,458,694 to Sollish, et al., entitled "Apparatus and Method for Detection of Tumors in Tissue," relates to an apparatus for detecting tumors in human breast, based on the dielectric constants of localized regions of the breast tissue. The apparatus includes a probe, including a plurality of elements. The apparatus further includes means for applying an AC signal to the tissue, means for sensing dielectric properties at each of the probe elements at different times, and signal processing circuitry, coupled to the sensing means, for comparing the dielectric properties sensed at the different times. The apparatus thus provides an output of the dielectric constants of localized regions of breast tissue associated with the probe.

Similarly, U.S. Patent 4,291,708 to Frei, et al., entitled "Apparatus and Method for Detection of Tumors in Tissue," relates to apparatus for detecting tumors in human breast tissue, by the dielectric constants of a plurality of localized regions of human breast tissue.

U.S. Patents 6,308,097; 6,055,452; and 5,810,742 to Pearlman, A. L., entitled "Tissue Characterization Based on Impedance Images and on Impedance Measurements," describe apparatus for aiding in the identification of tissue type for an anomalous tissue in an impedance image. The device comprises: means for providing a polychromic emmitance map of a portion of the body; means for determining a plurality of polychromic measures from the portion of the body; and a display for indicating the tissue type based on the plurality of polychromic measures.

Tissue characterization by optical fluorescence spectroscopy: When a sample of large molecules is irradiated, for example, by laser light, it will absorb radiation, and various levels will be excited. Some of the excited states will revert back substantially to the previous state, by elastic scattering, and some energy will be lost in internal conversion, collisions and other loss mechanisms. However, some excited states will create fluorescent radiation which, due to the distribution of states, will give a characteristic wavelength distribution.

Some tumor-marking agents give well-structured fluorescence spectra, when irradiated by laser light. In particular, hematoporphyrin derivatives (HPD), give a well-structured fluorescence spectrum, when excited in the Soret band, at around 405 nm. The fluorescence spectrum shows typical peaks at about 630 and 690 nm, superimposed in practice on more unstructured tissue autofluorescence. Other useful tumor-marking agents are dihematoporphyrin ether/ester (DHE), hematoporphyrin (HP), polyhematoporphyrin ester (PHE), and tetrasulfonated phthalocyanine (TSPC), when irradiated at 337 nm ($N_2$ laser).

U.S. Patent 5,115,137 to Andersson-Engels, et al., entitled "Diagnosis by Means of Fluorescent Light Emission from Tissue," relates to improved detection of properties of tissue by means of induced fluorescence of large molecules. The tissue character may then be evaluated from the observed large-molecule spectra. According to U.S. Patent 5,115,137, the spectrum for tonsil cancer is clearly different from that of normal mucosa, due to endogenous porphyrins.

U.S. Patent 6,258,576 to Richards-Kortum, et al., entitled "Diagnostic Method and Apparatus for Cervical Squamous Intraepithelial Lesions In Vitro and In Vivo Using Fluorescence Spectroscopy," relates to the use of multiple illumination wavelengths in fluorescence spectroscopy for the diagnosis of cancer and precancer, for example, in the cervix. In this manner, it has been possible to (i) differentiate normal or inflamed tissue from squamous intraepithelial lesions (SILs) and to (ii) differentiate high grade SILs from non-high grade SILs. The detection may be performed in vitro or in vivo. Multivariate statistical analysis has been employed to reduce the number of fluorescence excitation-emission wavelength pairs needed to re-develop algorithms that demonstrate a minimum decrease in classification accuracy. For example, the method of the aforementioned patent may comprise illuminating a tissue sample with electromagnetic radiation wavelengths of about 337 nm, 380 nm and 460 nm, to produce fluorescence; detecting a plurality of discrete emission wavelengths from the fluorescence; and calculating from the emission wavelengths a probability that the tissue sample belongs in particular tissue classification.

Commonly owned U.S. Patent Application 2003/01383786 to Hashimshony, entitled "Method and Apparatus for Examining Tissue for Predefined Target Cells, Particularly Cancerous Cells, and a Probe Useful for Such Method and Apparatus," teaches a method, apparatus, and probe for examining tissue and characterizing its type according to measured changes in optical characteristics of the examined tissue. In a preferred embodiment of this method the tissue to be examined is subject to a contrast agent containing small particles of a physical element conjugated with a biological carrier selectively bindable to the target cells. Additionally, energy pulses are applied to the examined tissue, and the changes in impedance and/or the optical characteristics produced by the applied energy pulses are detected and utilized for determining the presence of the target cells in the examined tissue. Furthermore, in a preferred embodiment, the applied energy pulses include laser pulses, and the physical element conjugated with a biological carrier is a light-sensitive semiconductor having an impedance which substantially decreases in the presence of light. Moreover, the same probe used for detecting the targeted cells may also be used for destroying the cells so targeted.

Tissue characterization by optical reflectance spectroscopy: The application of optical reflectance spectroscopy for tissue characterization is described, for example, in http://www.sbsp-limb.nichd.nih.gov/html/spectroscopy.html, downloaded on March 15, 2005, disclosing an optical reflectance spectroscopy (ORS) device for measuring the thickness of the epithelial layer, and an evaluation technique based on oblique angle reflectance spectroscopy, that allows assessment of the scattering and absorption properties of the epithelium and stroma, thus providing information on chronic oral epithelial tissue inflammation, which is considered a potential diagnostic precursor to oral cancer.

Additionally, Tomatis, A., et al., studied reflectance images of 43 pigmented lesions of the skin (18 melanomas, 17 common melanocytic naevi and eight dysplastic naevi). Reflectance images were acquired by a telespectrophotometric system and were analyzed in the spectral range from 420 to 1040 nm, to discriminate melanoma from benign melanocytic entities. Different evaluations were carried out considering the whole spectrum, the visible and the near infrared. A total of 33 (76.7%) lesions were correctly diagnosed by the telespectrophotometric system, compared with 35 (81.4%) correct clinical diagnoses. Reflectance in the infrared band appears diagnostically relevant.

Tissue characterization by magnetic resonance (MR): Magnetic resonance is based on the absorption and emission of energy in the radio frequency range of the electromagnetic spectrum, by nuclei having unpaired spins. Magnetic Resonance Imaging (MRI) is based on the imaging of the absorption and emission of energy in the radio frequency range of the electromagnetic spectrum, by nuclei having unpaired spins.

Conventional MRI utilizes a large-apparatus, for whole body imaging, having:

i. a primary magnet, which produces the $B_0$ field for the imaging procedure;
ii. gradient coils for producing a gradient in $B_0$;
iii. an RF coil, for producing the $B_1$ magnetic field, necessary to rotate the spins by 90° or 180° and for detecting the MR signal; and
iv. a computer, for controlling the components of the MR imager.

Generally, the magnet is a large horizontal bore superconducting magnet, which provides a homogeneous magnetic field in an internal region within the magnet. A patient or object to be imaged is usually positioned in the homogeneous field region located in the central air gap for imaging. A typical gradient coil system comprises an anti-Helmholtz type of coil. These are two parallel ring-shaped coils, around the z axis. Current in each of the two coils flows in opposite directions creating a magnetic field gradient between the two coils.

The RF coil creates a $B_1$ field, which rotates the net magnetization in a pulse sequence. The RF coils may be: 1) transmit and receive coils, 2) receive only coils, or 3) transmit only coils.

As described hereinabove, the MRI relies on a magnetic field in an internal region within the magnet. As such, it is unsuitable as a handheld probe or an endoscopic probe, because the tissue to be imaged has to be in the internal region of the imager.

However, U.S. Patent 5,572,132 to Pulyer, et al., entitled "MRI Probe for External Imaging," describes an MRI spectroscopic probe having an external background magnetic field $B_0$ (as opposed to the internal background magnetic field of the large horizontal bore superconducting magnet). Thus, an MRI catheter for endoscopical imaging of tissue of the artery wall, rectum, urinal tract, intestine, esophagus, nasal passages, vagina and other biomedical applications may be constructed. The probe comprises (i) a miniature primary magnet having a longitudinal axis and an external surface extending in the axial direction, and (ii) an RF coil surrounding and proximal to the surface. The primary magnet is structured and configured to provide a symmetrical, preferably cylindrically shaped, homogeneous field external to the surface of the magnet. The RF coil receives NMR signals from excited nuclei. For imaging, one or more gradient coils are provided to spatially encode the nuclear spins of nuclei excited by an RF coil, which may be the same coil used for receiving NMR signals, or another RF coil.

Additionally, commonly owned US Patent Application 2005/0021019 to Hashimshony, et al., entitled "Method and Apparatus for Examining Substance, Particularly Tissue, to Characterize its Type," describes a method and apparatus for examining a substance volume to characterize its type, by: applying a polarizing magnetic field through the examined substance; applying RF pulses locally to the examined substance volume such as to invoke electrical impedance (EI) response signals corresponding to the electrical impedance of the substance, and magnetic resonance (MR) response signals corresponding to the MR properties of the substance; detecting the EI and MR response signals; and utilizing the detected response signals for characterizing the examined substance volume type.

Contrast agents may be used in conjunction with MRI. For example, U.S. Patent 6,315,981 to Unger, entitled "Gas Filled Microspheres as Magnetic Resonance Imaging Contrast Agents, describes the use of gas filled microspheres as contrast agents for MRI.

Additionally, US Patent 6,747,454 to Belt, entitled "Array of Coils for Use in Imaging the Vasculature of a Patient," describes an array of coils, comprising first and second pluralities of coil pairs which are deployed longitudinally along anterior and posterior surfaces, respectively, of a patient. The first and second plurality of coil pairs receive magnetic resonance signals from the anterior and posterior surfaces, respectively. Means are provided for laterally isolating the first and second loops of each pair relative to each other. Means are provided for longitudinally isolating the coil pairs relative to each other. Means also vertically isolate the coil pairs of the first plurality from those of the second plurality. The magnetic resonance signals are utilized in imaging the vasculature of a patient.

Furthermore, US patent 6,677,755 to Belt, et al., entitled "Circuit for Selectively Enabling and Disabling Coils of a Multi-Coil Array," describes a circuit used to selectively enable and disable n-coils. The circuit includes n-drivers powered by a current source. Each n-driver includes a pair of FETs disposed such that a gate of one FET is connected to a gate of the other FET to form a common gate node thereat. The n-drivers are disposed in a totem-pole configuration. The first FET of a first of the n-drivers has (A) a drain linked to a ground and to an end of a first of the n-coils and (B) a source linked to a drain of the first FET of a second of the n-drivers and to an end of a second of the n-coils. The other FET of the first of the n-drivers has (A) a source linked to an opposite end of the first of the n-coils and (B) a drain linked to the end of the second of the n-coils and to the source of the first FET of the first of the n-drivers. The first FET of the second of the n-drivers also has a source linked to a drain of the first FET of a successive n-driver and to an end of a successive n-coil. The other FET of the second of the n-drivers also has (A) a source linked to an opposite end of the second of the n-coils and (B) a drain linked to the end of the successive n-coil and to the source of the first FET of the second of the n-drivers. This continues until the first FET and the other FET of an nth of the n-drivers are likewise disposed in the totem-pole configuration of the n-drivers, with a source and a drain of the first FET and the other FET, respectively, of the nth of the n-drivers being connected to the current source. Each of the n-drivers is used to operate a corresponding one of the n-coils by being responsive at its common gate node (i) to a coil disable signal by activating the first FET thereof and deactivating the other FET thereof thereby not only drawing current away from and thus disabling the corresponding coil but also allowing the current to flow through the first FET and thus to be available as a source of current to a successive one of the n-drivers and (ii) to a coil enable signal by deactivating the first FET thereof and activating the other FET thereof thereby allowing the current not only to flow serially through the corresponding coil and the other FET thus enabling the corresponding coil but also to be available as a source of current to the successive one of the n-drivers.

Tissue characterization by magnetic resonance spectroscopy (MRS): In MRS, spectroscopic NMR data is obtained from the examined area. Thus the biochemical information obtained from MRS can be interpreted in relation to a defined anatomical location, and images of metabolite distributions can be generated. MRS can be used to identify surrogate biochemical markers of cellular transformation, thus differentiating benign tumors from malignant ones, and identifying different tumor types. Prognostic and diagnostic information is derived from the spectrum of malignant tumors (Breast Cancer Res., 2001, 3:36-40).

Tissue characterization by radioactive emission: Radioactive-emission imaging relies on the fact that, in general, pathologies, such as malignant tumors and inflammations, display a level of activity different from that of healthy tissue. Thus, radiopharmaceuticals, which circulate in the blood stream, are picked up by the active pathologies to a different extent than by the surrounding healthy tissue; in consequence, the pathologies are operative as radioactive-emission sources and may be detected by radioactive-emission imaging.

The pathological feature may appear as a concentrated source of high radiation, or a hot region, as may be associated with a tumor, or as a region of low-level radiation, which is nonetheless above the background level, as may be associated with carcinoma. Additionally, a reversed situation is possible. Dead tissue has practically no pick up of radiopharmaceuticals, and is thus operative as a region of little radiation, or a cold region, below the background level. Thus radiopharmaceuticals may be used for identifying active pathologies as well as dead tissue, and the image that is constructed is generally termed, a "functional image."

The mechanism of localization of a radiopharmaceutical depends on various processes in the organ of interest, such as antigen-antibody reactions, physical trapping of particles, receptor site binding, removal of intentionally damaged cells from circulation, and transport of a chemical species across a cell membrane and into the cell by a normally operative metabolic process. A summary of the mechanisms of localization by radiopharmaceuticals is found in http://www.lunis.luc.edu/nucmed/tutorial/radpharm/i.htm.

The particular choice of a radionuclide for labeling antibodies depends upon the chemistry of the labeling procedure and the isotope nuclear properties, such as the number of gamma rays emitted; their respective energies; the emission of other particles, such as beta or positrons; the isotope half-life; and the existence of different isotopes of identical chemistry but having different half-lives (e.g., $I^{131}$ and $I^{133}$). The usual preferred emission for medical applications is that of gamma rays. However, beta and positron radiation may also be detected, and are of particular relevance in PET imaging.

The sensor may be a room temperature, solid-state CdZnTe (CZT) detector, configured as a single-pixel or a multi-pixel detector. Alternatively, the detector may be another solid-state detector such as CdTe, HgI, Si, Ge, or the like; a scintillation detector, such as NaI(Tl), LSO, GSO, CsI, CaF, or the like; or a combination of scintillation materials and photodiode arrays.

Two technologies of computed tomography for radioactive emission are known.

i. Single photon emission computed tomography (SPECT), in which single radioactive emission events are detected around a body. The detection of a large number of photons may be used to form a three-dimensional functional image and thus identify the source of the radiation.

ii. Positron emission tomography (PET), in which a positron is emitted from the radioactive isotope. Upon its interaction with an electron, annihilation occurs, and the two photons produced by the annihilation travel in opposite directions. Their detection by coincidence counting identifies an exact path upon which the annihilation took place. Again, the detection of a large number of photons may be used to form a three-dimensional functional image and identify the source of the radiation, especially using the fact that, in PET, the photon paths for coincidence counts are known.

Attenuation by the surrounding tissue introduces a certain error.

Various radiopharmaceuticals can be synthesized to target specific molecules present in the target tissue cells, such as, for example, [$^{18}$F] FDG (fluorodeoxyglucose), or an antibody fragment labeled with [$^{64}$Cu]. Others may be found in http://www.crump.ucla.edu/software/lpp/radioisotopes/tracers.html. Additional details and descriptions may be found in Breast Cancer Res. 2001, 3:28-35.

Tissue characterization by temperature imaging: Temperature imaging for locating and detecting neoplastic tissue has been known since the 1950's, when it was discovered that the surface temperature of skin in the area of a malignant tumor exhibited a higher temperature than that expected of healthy tissue. Thus, by measuring body skin temperatures, it became possible to screen for the existence of abnormal body activity such as cancerous tumor growth. With the development of liquid crystals and methods of forming temperature responsive chemical substrates, contact thermometry became a reality along with its use in medical applications. Devices employing contact thermometry could sense and display temperature changes through indicators which changed colors, either permanently or temporarily, when placed in direct physical contact with a surface such as skin, reflecting a temperature at or near the point of contact. An abnormal reading would alert a user to the need for closer, more detailed examination of the region in question. However, the art in this area has been directed primarily at sensing and displaying temperatures on exterior skin surfaces.

US Patent 3,830,224 to Vanzetti, et al., discloses the placement of temperature responsive, color changing liquid crystals at various points in a brassiere for the purpose of detecting the existence of breast cancer.

US Patent RE 32,000 to Sagi, entitled "Device for Use in Early Detection of Breast Cancer," discloses a device comprising a flexible, heat-conductive web, preferably in the form of a disc-shaped patch having an adhesive layer on one side thereof and a peelable layer removably secured thereto by the adhesive layer. On the other side thereof, the device comprises an array of spaced-apart indicators, each of the indicators comprising a dye or a pigment and a temperature sensitive substance (crystalline organic chemical) which melts at a relatively precise temperature which is approximately 0.5 degree F different from the adjacent indicator. As many indicators are used as are necessary to cover the desired temperature range. The device is incorporated into the breast-receiving cups of a brassiere and mirror image quadrants of the two breasts are scanned and the device is visually examined to determine the number of indicators which have displayed a change in color, thus apprising the person of the existence of abnormality in the mammary tissue.

U.S. Patent 6,135,968 to Brounstein, entitled "Differential Temperature Measuring Device and Method," describes a device and method for sensing temperatures at internal body locations non-surgically accessible only through body orifices. The device is particularly useful in medical applications such as screening for cancer and other abnormal biological activity signaled by an increase in temperature at a selected site. As applied to prostate examinations, the device is temporarily, adhesively affixed to a user's fingertip or to a mechanical probe. In the preferred embodiment, the device includes two temperature-sensing elements, which may include a plurality of chemical indicators. Each indicator changes color in response to detection of a predetermined particular temperature. When properly aligned and installed, the first element is located on the palmar surface of the fingertip while the second element is located on the dorsal surface of the fingertip. After an examination glove has been donned over the fingertip carrying the device, a prostate examination is performed during which the first element is brought into constant but brief contact with the prostate region and the second element is similarly, simultaneously brought into contact with a dermal surface opposing the prostate region. Upon withdrawal of the fingertip from the rectum and removal of the glove, the two temperature sensing elements may be visually examined in order to determine the temperatures detected by each one. A significant difference in observed temperatures indicates the possibility of abnormal biological activity and the need for further diagnostic or medical procedures.

Tissue characterization using biosensors: Biosensors may be of catalytic type such as integrated enzymes, cellular organelles, tissues or whole microorganisms with transducers that convert a biological response into a digital electronic signal. The principal transducers used are electrochemical, optical, or thermometric. Biosensors may also be of affinity type. Affinity biosensors deliver information about the binding of antibodies to antigens, cell receptors to their ligands, and DNA and RNA to nucleic acid with a complementary sequence. Still, additional types are fully integrated biochip devices that perform as micro bio-reactors. All types can be used in high-density arrays of bio-molecular sensors. Some of these sensors are further discussed in:

(i) Enzyme and Microbial Biosensors: Techniques and Protocols, A. Mulchandani & K. R. Rogers (Humana Press, 1998);
(ii) Affinity Biosensors: Techniques and Protocols, A. Mulchandani & K. R. Rogers (Humana Press, 1998);
(iii) Journal: Biosensors & Bioelectronics:

a. Volume 20, Issue 8, Pages 1459-1695 (15 February 2005);
b. Volume 20, Issue 6, Pages 1029-1259 (15 December 2004);
c. Volume 20, Issue 5, Pages 917-1028 (15 November 2004);
d. Volume 20, Issue 1, Pages 1-142 (30 July 2004);

e. Volume 20, Issue 12, Pages 2387-2593 (15 June 2005);

(iv) Journal: Sensors & Actuators B (chemical):

a. Volume 103, Issues 1-2, Pages 1-473 (29 September 2004);
b. Volume 102, Issue 1, Pages 1-177 (September 2004); and
c. Volume 106, Issue 1, Pages 1-488 (29 April 2005).

Tissue characterization using chemical sensors: Chemical sensors detect the presence of various types of chemical compounds and states. These include, for example, ions, such as, but not limited to, Na and K; dissolved gases, such as, but not limited to, oxygen and carbon dioxide; and sensors for determining Ph of solution.
Some of these sensors are further discussed in:

(i) Sensors: A Comprehensive Survey. Volume 2: Chemical and Biochemical Sensors, Part I, W. Gopel, J. Hesse, & J. N. Zemel (VCH, 1991);
(ii) Sensors: A Comprehensive Survey. Volume 3: Chemical and Biochemical Sensors, Part II, W. Gopel, J. Hesse, & J. N. Zemel (VCH, 1992); and
(iii) Journal: Sensors & Actuators B (Chemical):

a. Volume 103, Issues 1-2, Pages 1-473 (29 September 2004);
b. Volume 102, Issue 1, Pages 1-177 (September 2004);
c. Volume 106, Issue 1, Pages 1-488 (29 April 2005); and
d. Volume 108, Issues 1-2, Pages 1-1000 (22 July 2005).

Tissue characterization using mechanical sensors: Mechanical sensors measure a physical property of the tissue in contact with the sensor. One example of a mechanical sensor uses tactile sensing that measures the pressure sensed on the sensor surface. An optical tactile sensor having a transparent elastic tactile portion has been taught in US Patent 6,909,084 to Tachi and Kajimoto. This is an optical tactile sensor with a tactile section and imaging means, the tactile section comprising a transparent elastic body and a plurality of groups of markers provided inside the elastic body, each marker group made up of a number of colored markers, with markers making up different marker groups having different colors for each group, and behavior of the colored markers when an object touches the elastic body being photographed by the imaging means. Preferably the marker groups have mutually different spatial arrangements. Furthermore, mechanical sensors are discussed in: Sensors: A Comprehensive Survey, Volume 7: Mechanical Sensors, W. Gopel, J. Hesse, & J. N. Zemel (VCH, 1994).
It will be appreciated that the method, in accordance with some embodiments of the present invention may be adapted for human tissue and for animal tissue.
It will be appreciated that the probes according to embodiments of the present invention may be applied extracorporeally, to the skin. Alternatively, they may be applied to subcutaneous tissue, during open surgery. It will be appreciated that the probes according to embodiments of the present invention may be inserted intracorporeally, for a minimally invasive procedure having an incision of, for example, no greater than about 3 centimeters. Alternatively, they may be inserted into a body lumen.
It is expected that during the life of this patent many relevant broad-band sensors for tissue characterization will be developed and the scope of the term broad-band sensor for tissue characterization is intended to include all such new technologies a priori.
As used herein the terms "about" and "substantially" refer to $\pm$ 20 %.
It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.
Although the invention has been described in conjunction with specific embodiments thereof, it is evident that many alternatives, modifications and variations will be apparent to those skilled in the art. Accordingly, it is intended to embrace all such alternatives, modifications and variations that fall within the broad scope of the appended claims.

## Claims

1.  A device, comprising:

an element (20), which defines a rigid surface (22) of a linear cross-section, configured to make contact with a substance (44);
at least one sensor (24), in physical contact with the rigid surface; and
a mechanism (19), adapted for applying a force to the substance, for stretching the substance against the rigid surface, thus achieving effective contact between the substance and the rigid surface, **characterized in that**:
the line of force is at an acute angle with the rigid surface;
the at least one sensor (24) comprises a plurality of sensors;
the element (20) defines a curvature for obtaining three-dimensional information; and
the plurality of sensors (24) including at least two sensors, arranged along the curvature, each defining a viewing angle, the at least two sensors sharing a portion of their viewing angles so as to obtain three-dimensional information.

2. The device of claim 1, wherein the stretching further includes stretching and pushing, or wherein the acute angle is between 30 degrees and 60 degrees, or wherein the effective contact is a contact level of at least 95%.

3. The device of claim 1, wherein the at least one sensor includes at least two different types of sensors.

4. The device of claim 1, wherein the three-dimensional information includes small-scale computerized tomography.

5. The device of claim 1, wherein the mechanism (19) is suction, or is tweezer-like (13), or exerts physical pressure (17) on the substance.

6. A method of substance characterization, comprising:

providing a substance characterization device, which comprises:

an element (20), which defines a rigid surface (22) of a linear cross-section, configured to make contact with a substance (44);
a plurality of sensors (24), in physical contact with the rigid surface (22); and
a mechanism (19), adapted for applying a force to the substance (44), the line of force being at an acute angle with the rigid surface, for stretching the substance (44) against the rigid surface (22), thus achieving effective contact between the substance and the rigid surface, wherein the element defines a curvature for obtaining three-dimensional information, and further wherein the plurality of sensors (24) includes at least two sensors, arranged along the curvature, each defining a viewing angle, the at least two sensors sharing a portion of their viewing angles so as to obtain three-dimensional information;

the method further comprising applying the force to the substance (44), the line of force being at an acute angle with the rigid surface (22), for stretching the substance (44) against the rigid surface (22), thus achieving effective contact between the substance (44) and the rigid surface (22), thereby to condition the substance for characterization by the plurality of sensors (24).

**Patentansprüche**

1. Vorrichtung, umfassend:

ein Element (20), das eine starre Oberfläche (22) eines linearen Querschnitts definiert, das konfiguriert ist, um Kontakt mit einer Substanz (44) herzustellen;
wenigstens einen Sensor (24) in physischem Kontakt mit der starren Oberfläche; und
einen Mechanismus (19), der eingerichtet ist, um eine Kraft auf die Substanz aufzubringen, um die Substanz gegen die starre Oberfläche zu dehnen, wodurch ein wirksamer Kontakt zwischen der Substanz und der starren Oberfläche erreicht wird, **dadurch gekennzeichnet, dass**:

die Kraftlinie in einem spitzen Winkel zur starren Oberfläche verläuft;
der wenigstens eine Sensor (24) eine Vielzahl von Sensoren umfasst;
das Element (20) eine Krümmung zum Erhalten dreidimensionaler Informationen definiert; und
wobei die Vielzahl von Sensoren (24) wenigstens zwei Sensoren beinhaltet, die entlang der Krümmung angeordnet sind, wobei jeder einen Betrachtungswinkel definiert, wobei die wenigstens zwei Sensoren einen Abschnitt ihrer Betrachtungswinkel gemeinsam nutzen, um dreidimensionale Informationen zu erhalten.

2. Vorrichtung nach Anspruch 1, wobei das Dehnen ferner Dehnen und Schieben beinhaltet oder wobei der spitze Winkel zwischen 30 und 60 Grad beträgt oder wobei der wirksame Kontakt eine Kontaktstärke von wenigstens 95 % aufweist.

3. Vorrichtung nach Anspruch 1, wobei der wenigstens eine Sensor wenigstens zwei verschiedene Arten von Sensoren beinhaltet.

4. Vorrichtung nach Anspruch 1, wobei die dreidimensionalen Informationen eine Computertomographie im kleinen Maßstab beinhaltet.

5. Vorrichtung nach Anspruch 1, wobei es sich bei dem Mechanismus (19) um ein Saugen oder eine zupfartige Aktivität (13) oder um ein Aufbringen eines physikalischen Drucks (17) auf die Substanz handelt.

6. Verfahren zur Substanzcharakterisierung, umfassend: Bereitstellen einer Vorrichtung zur Substanzcharakterisierung, die umfasst:

ein Element (20), das eine starre Oberfläche (22) eines linearen Querschnitts definiert, das konfiguriert ist, um einen Kontakt mit einer Substanz (44) herzustellen;
eine Vielzahl von Sensoren (24) in physischem Kontakt mit der starren Oberfläche (22); und
einen Mechanismus (19), der eingerichtet ist, um eine Kraft auf die Substanz (44) aufzubringen, wobei die Kraftlinie in einem spitzen Winkel zu der starren Oberfläche verläuft, um die Substanz (44) gegen die starre Oberfläche (22) zu dehnen und dadurch einen wirksamen Kontakt zwischen der Substanz und der starren Oberfläche zu erhalten, wobei das Element eine Krümmung definiert, um dreidimensionale Informationen zu erhalten, und wobei die Vielzahl von Sensoren (24) ferner wenigstens zwei Sensoren beinhaltet, die entlang der Krümmung angeordnet sind, die jeweils einen Betrachtungswinkel definieren, wobei die wenigstens zwei Sensoren einen Abschnitt ihrer Betrachtungswinkel gemeinsam nutzen, um dreidimensionale Informationen zu erhalten;
wobei das Verfahren ferner Aufbringen der Kraft auf die Substanz (44) umfasst, wobei die Kraftlinie in einem spitzen Winkel zu der starren Oberfläche (22) verläuft, um die Substanz (44) gegen die starre Oberfläche (22) zu dehnen und so einen wirksamen Kontakt zwischen der Substanz (44) und der starren Oberfläche (22) zu erhalten, um dadurch die Substanz zur Charakterisierung durch die Vielzahl von Sensoren (24) aufzubereiten.

**Revendications**

1. Dispositif comprenant :

un élément (20) qui délimite une surface rigide (22) d'une section transversale linéaire, conçue pour entrer en contact avec une substance (44) ;
au moins un capteur (24), en contact physique avec la surface rigide ; et
un mécanisme (19), conçu pour appliquer une force à la substance, pour étirer la substance contre la surface rigide, permettant d'établir ainsi un contact efficace entre la substance et la surface rigide, **caractérisé en ce que** :

la ligne de force forme un angle aigu avec la surface rigide ;
l'au moins un capteur (24) comprend une pluralité de capteurs ;
l'élément (20) délimite une courbure servant à obtenir des informations tridimensionnelles ; et
la pluralité de capteurs (24) comprenant au moins deux capteurs, disposés le long de la courbure, chacun délimitant un angle de vision, les au moins deux capteurs partageant une partie de leurs angles de vision afin d'obtenir des informations tridimensionnelles.

2. Dispositif selon la revendication 1, dans lequel l'étirement comprend en outre l'étirement et la poussée, ou dans lequel l'angle aigu est compris entre 30 degrés et 60 degrés, ou dans lequel le contact effectif est un niveau de contact d'au moins 95 %.

**3.** Dispositif selon la revendication 1, dans lequel l'au moins un capteur comprend au moins deux types différents de capteurs.

**4.** Dispositif selon la revendication 1, dans lequel les informations tridimensionnelles comprennent une tomodensitométrie à petite échelle.

**5.** Dispositif selon la revendication 1, dans lequel le mécanisme (19) est à ventouse, ou est semblable à des pinces (13), ou exerce une pression physique (17) sur la substance.

**6.** Procédé de caractérisation de substance, comprenant : la fourniture d'un dispositif de caractérisation de substance, qui comprend :

un élément (20) qui délimite une surface rigide (22) d'une section transversale linéaire, conçue pour entrer en contact avec une substance (44) ;
une pluralité de capteurs (24), en contact physique avec la surface rigide (22) ; et
un mécanisme (19), conçu pour appliquer une force à la substance (44), la ligne de force formant un angle aigu avec la surface rigide, pour étirer la substance (44) contre la surface rigide (22), permettant d'obtenir ainsi un contact efficace entre la substance et la surface rigide, l'élément délimitant une courbure servant à obtenir des informations tridimensionnelles, et en outre dans lequel la pluralité de capteurs (24) comprend au moins deux capteurs, disposés le long de la courbure, chacun délimitant un angle de vision, les au moins deux capteurs partageant une partie de leurs angles de vision afin d'obtenir des informations tridimensionnelles ;
le procédé comprenant en outre l'application de la force à la substance (44), la ligne de force formant un angle aigu avec la surface rigide (22) pour étirer la substance (44) contre la surface rigide (22), permettant d'obtenir ainsi un contact efficace entre la substance (44) et la surface rigide (22), pour préparer ainsi la substance à la caractérisation par la pluralité de capteurs (24).

Figure 1A

Figure 1B

Figure 1C

Figure 1D

Figure 1E

Figure 1F

Figure 1G

EP 1 919 273 B1

Cross-Sectional View
Figure 1H

**Figure 1I**

**Figure 1L**

**Figure 1N**

**Figure 1 J**

**Figure 1 K**

**Figure 1M**

EP 1 919 273 B1

20

Figure 1O  Figure 1P  Figure 1Q  Figure 1 R

20 F

α

22

Figure 1S  Figure 1T  Figure 1U  Figure 1V

EP 1 919 273 B1

Figure 2A

Figure 2B

EP 1 919 273 B1

Figure 2C

Figure 2D

32

**Figure 2E**

**Figure 3**

Figure 4A

**Figure 4B**

EP 1 919 273 B1

# Figure 5

90

**92**

arranging a sensor for the characterization of a soft
tissue on a rigid surface, having a linear cross section

**94**

applying a force to the soft tissue at a sharp angle to the rigid surface of the linear cross section,

thus stretching or stretching and pushing the soft tissue against the rigid surface,

and achieving a contact level of at least 95%,

forming an effective contact

between the sensor and the tissue

**96**

performing measurements by the at least one sensor

Figure 6A

Figure 6B

**Figure 6C**

Figure 6F

Figure 6E

Figure 6D

20A

**Figure 6G**

20A

D'

**Figure 6H**

20A

**Figure 6I**

20A

D

**Figure 6J**

20A

**Figure 6K**

20A

**Figure 6L**

20A

**Figure 6M**

20A

**Figure 6N**

EP 1 919 273 B1

# Figure 6O

130

## 132

arranging at least two sensors on a curved surface, so that the at least two sensors view the same volumetric region of the tissue

## 134

performing measurements

with the at least two sensors

## 136

analyzing the measurements to obtain three-dimensional information of the volumetric region

Fig. 7A

Cross-Sectional View
Fig. 7C

Fig. 7B

Fig. 8A

Fig. 8B

Fig. 8C

Fig. 9C

Fig. 9A

Fig. 9B

Fig. 10B

Fig. 10D

Fig. 10A

Fig. 10C

Fig. 11A          Fig. 11B

Figure 12A

Figure 12B

Fig. 13A                                    Fig. 13B

EP 1 919 273 B1

Fig. 13C

Fig. 13D

EP 1 919 273 B1

172

z

y

x

Fig. 14A

126

164

122

Fig. 14B

126

164

122

Fig. 14C

126

164

122

Fig. 14D

EP 1 919 273 B1

EP 1 919 273 B1

Fig. 15A

Fig. 15B

Fig. 15C

Fig. 15D

EP 1 919 273 B1

Fig. 16A

100

302

12

300

105

113

50

Fig. 16B

100

120

105

XY
Z
172

Fig. 16C

126

144

110

105

100

A

A

Fig. 16D

100

172

XY
Z

54

Fig. 17B

Fig. 17A

EP 1 919 273 B1

Fig. 17C

Fig. 17D

Fig. 17E

56

Figure 18A

Figure 18B

Figure 18C

EP 1 919 273 B1

Figure 19

EP 1 919 273 B1

Figure 20A

Figure 20B

Figure 20C

EP 1 919 273 B1

Figure 21A

Figure 21B

EP 1 919 273 B1

EP 1 919 273 B1

Figure 22

Figure 23

Figure 24A

Figure 24B

EP 1 919 273 B1

EP 1 919 273 B1

Figure 25A

Figure 25B

Figure 25C

Figure 25D

63

Figure 26A

EP 1 919 273 B1

Figure 26B

EP 1 919 273 B1

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 5927284 A, Borst
- US 5727569 A, Benetti
- US 6728565 B, Wendlandt
- US 6090041 A, Clark
- US 6695782 B, Ranucci
- US 6500112 B, Khouri
- WO 2006116091 A, Richard J. Davies
- US 60665842 B
- US 6813515 B
- WO 03060462A A
- US 10298196 B
- US 20050021019 A, Hashimshony
- US 5572132 A, Pulyer
- US 6280704 B, Schutt
- US 60641081 B
- US 4458694 A, Sollish
- US 4291708 A, Frei
- US 6308097 B
- US 6055452 A
- US 5810742 A, Pearlman, A. L.
- US 5115137 A, Andersson-Engels
- US 6258576 B, Richards-Kortum
- US 200301383786 A, Hashimshony
- US 6315981 B
- US UNGER A
- US 6747454 B
- US BELT A
- US 6677755 B, Belt
- US 3830224 A, Vanzetti
- US RE32000 E, Sagi
- US 6135968 A, Brounstein
- US 6909084 B, Tachi and Kajimoto

## Non-patent literature cited in the description

- **RAJSHEKHAR, V.** Continuous Impedance Monitoring During CT-Guided Stereotactic Surgery: Relative Value in Cystic and Solid Lesions. *the British Journal of Neurosurgery,* 1992, vol. 6, 439-444
- *Breast Cancer Res.,* 2001, vol. 3, 36-40
- *Breast Cancer Res.,* 2001, vol. 3, 28-35
- **A. MULCHANDANI ; K. R. ROGERS.** Enzyme and Microbial Biosensors: Techniques and Protocols. Humana Press, 1998
- **A. MULCHANDANI ; K. R. ROGERS.** Affinity Biosensors: Techniques and Protocols. Humana Press, 1998
- *JOURNAL: BIOSENSORS & BIOELECTRONICS,* 15 February 2005, vol. 20 (8), 1459-1695
- *JOURNAL: BIOSENSORS & BIOELECTRONICS,* 15 December 2004, vol. 20 (6), 1029-1259
- *JOURNAL: BIOSENSORS & BIOELECTRONICS,* 15 November 2004, vol. 20 (5), 917-1028
- *JOURNAL: BIOSENSORS & BIOELECTRONICS,* 30 July 2004, vol. 20 (1), 1-142
- *JOURNAL: BIOSENSORS & BIOELECTRONICS,* 15 June 2005, vol. 20 (12), 2387-2593
- *JOURNAL: SENSORS & ACTUATORS B (CHEMICAL),* 29 September 2004, vol. 103 (1-2), 1-473
- *JOURNAL: SENSORS & ACTUATORS B (CHEMICAL),* September 2004, vol. 102 (1), 1-177
- *JOURNAL: SENSORS & ACTUATORS B (CHEMICAL),* 29 April 2005, vol. 106 (1), 1-488
- Sensors: A Comprehensive Survey. **W. GOPEL ; J. HESSE ; J. N. ZEMEL.** Chemical and Biochemical Sensors. VCH, 1991, vol. 2
- Sensors: A Comprehensive Survey. **W. GOPEL ; J. HESSE ; J ; N. ZEMEL.** Chemical and Biochemical Sensors. VCH, 1992, vol. 3
- *JOURNAL: SENSORS & ACTUATORS B (CHEMICAL),* 22 July 2005, vol. 108 (1-2), 1-1000
- Sensors: A Comprehensive Survey. **W. GOPEL ; J. HESSE ; J. N. ZEMEL.** Mechanical Sensors. VCH, 1994, vol. 7